(19) Europäisches Patentamt
European Patent Office
Office européen des brevets

(11) EP 4 679 130 A1

(12) **EUROPEAN PATENT APPLICATION**
published in accordance with Art. 153(4) EPC

(43) Date of publication:
**14.01.2026 Bulletin 2026/03**

(21) Application number: **24767083.9**

(22) Date of filing: **01.03.2024**

(51) International Patent Classification (IPC):
***G01S 7/41** (2006.01)* ***A61B 5/11** (2006.01)*
***G01S 13/34** (2006.01)* ***G01S 13/89** (2006.01)*

(52) Cooperative Patent Classification (CPC):
**A61B 5/11; G01S 7/02; G01S 7/41; G01S 13/34; G01S 13/89**

(86) International application number:
**PCT/JP2024/007887**

(87) International publication number:
**WO 2024/185715 (12.09.2024 Gazette 2024/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.03.2023 JP 2023034884**

(71) Applicant: **Kyocera Corporation**
**Kyoto-shi, Kyoto 612-8501 (JP)**

(72) Inventors:
- **MATSUE Yudai**
  **Kyoto-shi, Kyoto 612-8501 (JP)**
- **KURODA Jun**
  **Kyoto-shi, Kyoto 612-8501 (JP)**

(74) Representative: **Viering, Jentschura & Partner mbB**
**Patent- und Rechtsanwälte**
**Am Brauhaus 8**
**01099 Dresden (DE)**

(54) **ELECTRONIC DEVICE, METHOD FOR CONTROLLING ELECTRONIC DEVICE, AND PROGRAM**

(57) An electronic device is provided with a signal processing unit. The signal processing unit is configured to detect an object on the basis of a transmission signal transmitted as a transmission wave and a received signal received as a reflected wave resulting from the transmission wave being reflected by the object. The signal processing unit is configured to extract, from a result of estimating an angle, with respect to the electronic device, of a human body to be detected on the basis of the transmission signal and the received signal over a prescribed time, two-dimensional data having a time direction and a velocity direction that correspond to a distance between the electronic device and the human body, and extract a prescribed feature on the basis of the two-dimensional data.

FIG. 2

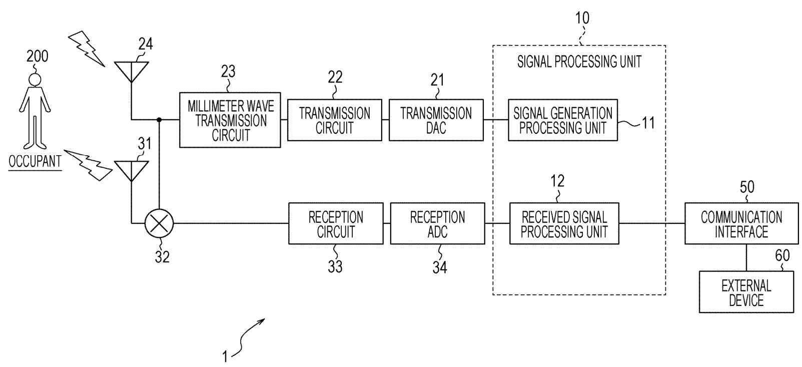




EP 4 679 130 A1

## Description

CROSS-REFERENCE TO RELATED APPLICATIONS

This application claims priority based on Japanese Patent Application No. 2023-34884 filed March 7, 2023, the entire disclosure of which is hereby incorporated by reference.

TECHNICAL FIELD

The present disclosure relates to an electronic device, a method of controlling an electronic device, and a program.

BACKGROUND OF INVENTION

In fields such as the automotive and related industries, for example, technologies for measuring values such as the distance between a vehicle and a given object are gaining importance. In particular, recent years have seen various research into radio detection and ranging (RADAR) technology, which measures values such as the distance to an obstacle or other object by transmitting radio waves, such as millimeter waves, and receiving reflected waves back from the object. The importance of technologies for measuring such distances and the like is expected to increase further with the development of technologies for assisting drivers with driving and technologies related to self-driving, in which driving is partially or fully automated.

Various technologies have been proposed to detect the presence or the like of a given object by receiving reflected waves of transmitted radio waves or the like reflecting off the object. As an example, Patent Literature 1 proposes a technology for detecting the number and position of occupants in the cabin of an automobile by using a radar unit provided in the cabin. Patent Literature 1 discloses a technology for detecting the status of an occupant in the cabin by using a transmitter/receiver located closest to the driver's seat from among the seats in the cabin. As another example, Non-Patent Literature 1 proposes a technology for detecting an occupant inside an automobile by using machine learning to process a detection signal from a millimeter-wave radar. Non-Patent Literature 1 discloses a technology for distinguishing the specific row in the cabin where an occupant is present.

CITATION LIST

PATENT LITERATURE

Patent Literature 1: Japanese Unexamined Patent Application Publication No. 2017-181225

NON PATENT LITERATURE

Non Patent Literature 1: ALIZADEH Mostafa, ABEDI Hajar, SHAKER George. "Low-cost low-power in-vehicle occupant detection with mm-wave FMCW radar" 2019, IEEE SENSORS. p. 1-4.

SUMMARY

In an embodiment, an electronic device is provided with a signal processing unit. The signal processing unit is configured to detect an object on the basis of a transmission signal transmitted as a transmission wave and a received signal received as a reflected wave resulting from the transmission wave being reflected by the object. The signal processing unit is configured to extract, from a result of estimating an angle, with respect to the electronic device, of a human body to be detected on the basis of the transmission signal and the received signal over a prescribed time, two-dimensional data having a time direction and a velocity direction that correspond to a distance between the electronic device and the human body, and extract a prescribed feature on the basis of the two-dimensional data.

In an embodiment, an electronic device is provided with a signal processing unit. The signal processing unit is configured to detect an object on the basis of a transmission signal transmitted as a transmission wave and a received signal received as a reflected wave resulting from the transmission wave being reflected by the object. The signal processing unit is configured to extract, from a result of estimating an angle, with respect to the electronic device, of a human body detected on the basis of the transmission signal and the received signal over a prescribed time, two-dimensional data having a time direction and a velocity direction that correspond to a distance between the electronic device and the human body, extract a prescribed feature on the basis of the two-dimensional data, and thereby determine whether a human is present or absent in the surroundings of the electronic device and/or a position where the human is present.

In an embodiment, a method of controlling an electronic device is provided. The method includes detecting an object on the basis of a transmission signal transmitted as a transmission wave and a received signal received as a reflected wave resulting from the transmission wave being reflected by the object. The method includes extracting, from a result of estimating an angle, with respect to the electronic device, of a human body to be detected on the basis of the transmission signal and the received signal over a prescribed time, two-dimensional data having a time direction and a velocity direction that correspond to a distance between the electronic device and the human body. The method includes extracting a prescribed feature on the basis of the two-dimensional data.

In an embodiment, a program causes an electronic device to execute a process. The process includes detecting an object on the basis of a transmission signal transmitted as a transmission wave and a received signal received as a reflected wave resulting from the transmission wave being reflected by the object. The process includes extracting, from a result of estimating an angle, with respect to the electronic device, of a human body to be detected on the basis of the transmission signal and the received signal over a prescribed time, two-dimensional data having a time direction and a velocity direction that correspond to a distance between the electronic device and the human body. The process includes extracting a prescribed feature on the basis of the two-dimensional data.

## BRIEF DESCRIPTION OF THE DRAWINGS

FIG. 1 is a diagram for describing how an electronic device according to an embodiment is used.
FIG. 2 is a function block diagram schematically illustrating a configuration of the electronic device according to an embodiment.
FIG. 3 is a diagram for describing the composition of a signal processed by an electronic device according to an embodiment.
FIG. 4 is a diagram for describing the processing of a signal by an electronic device according to an embodiment.
FIG. 5 is a diagram for describing the processing of a signal by an electronic device according to an embodiment.
FIG. 6 is a diagram for describing the processing of a signal by an electronic device according to an embodiment.
FIG. 7 schematically illustrates an example of the antenna arrangement and the operating principle of an antenna array of an electronic device according to an embodiment.
FIG. 8 illustrates an example of the antenna arrangement in an antenna array of an electronic device according to an embodiment.
FIG. 9 illustrates an example of an electronic device according to an embodiment installed inside an automobile.
FIG. 10 is a diagram illustrating an example of the position where an electronic device according to an embodiment is located inside an automobile and the positions of seats disposed inside the automobile.
FIG. 11 is a diagram illustrating an example of a result in which an occupant is detected by a radar sensor in the cabin of an automobile.
FIG. 12 is a flowchart for describing operations by an electronic device according to an embodiment.
FIG. 13 is a diagram for conceptually describing signal processing by an electronic device according to an embodiment.
FIG. 14 is a diagram for conceptually describing signal processing by an electronic device according to an embodiment.
FIG. 15 is a diagram for conceptually describing signal processing by an electronic device according to an embodiment.
FIG. 16 is a diagram illustrating an example of features generated by an electronic device according to an embodiment.
FIG. 17 is a diagram for conceptually describing calculations by an electronic device according to an embodiment.
FIG. 18 illustrates an example of a result of processing by an electronic device according to an embodiment.
FIG. 19 illustrates an example of a result of processing by an electronic device according to an embodiment.
FIG. 20 illustrates an example of a result of processing by an electronic device according to an embodiment.
FIG. 21 is a flowchart for describing operations by an electronic device according to an embodiment.

## DESCRIPTION OF EMBODIMENTS

The ability to detect the presence and the presence positions of occupants, including the driver, with good accuracy in an at least partially enclosed space, such as inside an automobile for example, by transmitting and receiving radio waves, such as millimeter waves for example, could be useful in a wide variety of fields. An objective of the present disclosure is to provide an electronic device, a method of controlling an electronic device, and a program with which the presence and the presence position of a human or the like can be detected with good accuracy by transmitting and receiving radio waves in an at least partially enclosed space. According to an embodiment, an electronic device, a method of controlling an electronic device, and a program with which the presence and the presence position of a human or the like can be detected with good accuracy by transmitting and receiving radio waves in an at least partially enclosed space can be provided. The following describes an embodiment in detail, with reference to the drawings.

In the present disclosure, an "electronic device" may be a device that operates on electric power. A "user" may be an entity (typically a human being) or an animal that uses a system and/or an electronic device according to an embodiment. The user may include an entity that, for example, monitors or observes a human being or other subject by using an electronic device according to an embodiment. The "subject" may be an entity (a human being or an animal, for example) to be monitored using an electronic device according to an embodiment. The user may also include the subject.

In an embodiment, an electronic device according can detect the presence and the presence position of, for example, an occupant, including the driver, in an at least a partially enclosed space such as, for example, the interior of an automobile where the electronic device is installed. Accordingly, anticipated situations in which an electronic device according to an embodiment is used may be, for example, inside a moving body, such as inside an automobile, where the electronic device is installed. The moving body inside which an electronic device according to an embodiment can be installed is not limited to an automobile or the like. For example, the moving body inside which an electronic device according to an embodiment is installed may be any of various kinds of moving bodies, such as self-driving cars, buses, trucks, taxis, ships, aircraft, helicopters, spacecraft, rockets, tractors and other agricultural equipment, snowplows, sanitation vehicles, police cars, and ambulances. Moving bodies such as automobiles included in the present disclosure are not limited by overall length, overall width, overall height, engine displacement, maximum occupancy, load capacity, or the like. For example, automobiles in the present disclosure include automobiles with an engine displacement greater than 660 cc and automobiles with an engine displacement of 660 cc or less, which are also referred to as light automobiles. Automobiles included in the present disclosure include automobiles that partly or fully use electricity for energy, and automobiles that use a motor.

Furthermore, anticipated situations in which an electronic device according to an embodiment is used are not necessarily limited to the inside of a moving body. For example, an electronic device according to an embodiment may also be installed indoors, such as inside a room. For example, an electronic device according to an embodiment may also be installed inside an office, inside a conference room, inside a storage room, inside a hospital room, inside a lavatory, inside a shop, inside a factory, or the like. Anticipated situations in which an electronic device according to an embodiment is used are not necessarily limited only to places where humans are present, and may also include places where animals other than humans are present. For example, in an embodiment, an electronic device may be installed inside a cage where a pet or the like is kept, inside a barn where livestock or the like is kept, or inside a container used for transporting animals.

In an embodiment, an electronic device may be installed in any moving body, and may also be installed in any stationary object. In an embodiment, an electronic device can transmit a transmission wave from a transmitting antenna to the surroundings of the electronic device. The transmitting antenna may be formed from multiple antennas. In an embodiment, an electronic device can receive, from a receiving antenna, a reflected wave resulting from the transmission wave being reflected. The receiving antenna may be formed from multiple antennas. The transmitting antenna and/or the receiving antenna may be provided in the electronic device, and may also be provided in a radar sensor, for example.

The following describes an electronic device according to an embodiment in detail, with reference to the drawings. First, an example of the detection of an object by an electronic device according to an embodiment will be described.

FIG. 1 is a diagram for describing an example of how an electronic device according to an embodiment is used. FIG. 1 illustrates an example of an electronic device provided with the functions of a sensor provided with a transmitting antenna and a receiving antenna according to an embodiment. In an embodiment, the electronic device may include functions based on frequency-modulated continuous-wave radar (FMCW) technology, for example.

As illustrated in FIG. 1, in an embodiment, an electronic device 1 may be provided with a transmission unit and a reception unit. As described later, the transmission unit may be provided with a transmitting antenna array 24. The reception unit may be provided with a receiving antenna array 31. Specific configurations of the electronic device 1, the transmission unit, and the reception unit will be described later. For simplicity, FIG. 1 schematically illustrates a situation in which the electronic device 1 is provided with the transmitting antenna array 24 and the receiving antenna array 31. The electronic device 1 may also include at least one other functional unit, as appropriate, such as at least a portion of a signal processing unit 10 (FIG. 2) included in the electronic device 1. The electronic device 1 may be provided with at least one other functional unit outside the electronic device 1, such as at least a portion of a signal processing unit 10 (FIG. 2) included in the electronic device 1. In FIG. 1, the electronic device 1 may be moving, but may also be stationary without moving.

The example illustrated in FIG. 1 illustrates in a simplified manner the transmission unit provided with the transmitting antenna array 24 and the reception unit provided with the receiving antenna array 31 in the electronic device 1. The electronic device 1 may also be provided with a plurality of transmission units and/or a plurality of reception units, for example. The transmission unit may be provided with a transmitting antenna array 24 formed from a plurality of transmitting antennas. The reception unit may be provided with a receiving antenna array 31 formed from a plurality of receiving antennas. The position where the transmission unit and/or reception unit are installed in the electronic device 1 is not limited to the position illustrated in FIG. 1, and may be another position, as appropriate. The number of transmission units and/or reception units may be any number equal to or greater than 1, according to various conditions (or requirements) such as the range and/or precision of heartbeat detection by the electronic device 1.

As described later, the electronic device 1 transmits an electromagnetic wave as a transmission wave from the transmitting antenna array 24. For example, if a given object (for example, the subject 200 illustrated in FIG. 1) is present in the surroundings of the electronic device 1, at least a portion of the transmission wave transmitted from the electronic device 1 is reflected by the object to become a reflected wave. Such a reflected wave is then received by the receiving antenna array 31 of the electronic device 1, for example, whereby the electronic device 1 can detect the subject as a target.

Typically, the electronic device 1 provided with the transmitting antenna array 24 may be a radio detection and ranging (RADAR) sensor that transmits and receives radio waves. However, the electronic device 1 is not limited to a radar sensor. In an embodiment, the electronic device 1 may also be, for example, a sensor based on light detection and ranging (LIDAR) technology, also known as laser imaging detection and ranging, which involves light waves. Sensors such as these can be configured to include a patch antenna or the like. Since technologies such as RADAR and LIDAR are already known, a detailed description may be simplified or omitted, as appropriate. In an embodiment, the electronic device 1 may also be a sensor based on a technology that detects objects by transmitting and receiving sonic or ultrasonic waves, for example.

The electronic device 1 illustrated in FIG. 1 receives from the receiving antenna array 31 a reflected wave of a transmission wave transmitted from the transmitting antenna array 24. With this arrangement, the electronic device 1 can detect a given subject 200 present within a given distance from the electronic device 1 as a target. For example, as illustrated in FIG. 1, the electronic device 1 can measure the distance L between the electronic device 1 and a given subject 200. The electronic device 1 can also measure the relative velocity between the electronic device 1 and a given subject 200. The electronic device 1 can also measure (estimate) the direction (angle of arrival $\theta$) from which the reflected wave from a given subject 200 arrives at the electronic device 1.

In FIG. 1, the XY plane may be defined as the plane substantially parallel to the ground, for example. In this case, the positive direction of the Z axis illustrated in FIG. 1 may indicate the vertically upward direction. In FIG. 1, the electronic device 1 may be located on a plane parallel to the XY plane. In FIG. 1, the subject 200 may be present on the ground substantially parallel to the XY plane, for example.

The subject 200 may be a human being or the like present in the surroundings of the electronic device 1, for example. The subject 200 may also be a living thing other than a human being, such as an animal, present in the surroundings of the electronic device 1, for example. As described above, the subject 200 may be moving, and may also be stopped or stationary. In the present disclosure, the object to be detected by the electronic device 1 includes inanimate things such as any object, as well as living things such as people, dogs, cats, horses, and other animals. The object to be detected by the electronic device 1 according to the present disclosure may also include a target, including a person, a thing, an animal, or the like, that is detected by radar technology. In the present disclosure, a target may include a person, a thing, an animal, or the like. The following description assumes that the object such as the subject 200 present in the surroundings of the electronic device 1 is a human being (or an animal). Hereinafter, the "subject 200" is also referred to as the "occupant 200", as appropriate. The occupant 200 may be a person appearing on a moving body such as an automobile in which the electronic device 1 is installed, for example. In the present disclosure, the target may also be the subject 200 above.

In FIG. 1, the ratio of the size of the electronic device 1 to the size of the subject 200 does not necessarily indicate the actual ratio. In FIG. 1, the transmitting antenna array 24 of the transmission unit and the receiving antenna array 31 of the reception unit are illustrated as being installed on the outside of the electronic device 1. However, in an embodiment, the transmitting antenna array 24 of the transmission unit and/or the receiving antenna array 31 of the reception unit may be installed at various positions in the electronic device 1. For example, in an embodiment, the transmitting antenna array 24 of the transmission unit and/or the receiving antenna array 31 of the reception unit may be installed inside the electronic device 1, and may not appear on the exterior of the electronic device 1.

The following describes a typical example in which the transmitting antenna of the electronic device 1 transmits radio waves in a frequency band such as millimeter waves (30 GHz or higher) or quasi-millimeter waves (for example, around 20-30 GHz). On the other hand, the transmitting antenna of the electronic device 1 may also transmit radio waves with a frequency bandwidth of 4 GHz, such as from 77 GHz to 81 GHz, for example.

FIG. 2 is a function block diagram schematically illustrating an example configuration of the electronic device 1 according to an embodiment. The following describes an example of the configuration of the electronic device 1 according to an embodiment.

Frequency-modulated continuous-wave radar (hereinafter referred to as FMCW radar) is often used to measure distances and the like using millimeter-wave radar. In FMCW radar, a transmission signal is generated by sweeping the frequencies of the radio waves to be transmitted. Accordingly, in a millimeter-wave FMCW radar that uses radio waves in the 79 GHz frequency band, for example, the frequencies of the radio waves to be used have a frequency bandwidth of 4 GHz, such as from 77 GHz to 81 GHz, for example. Radar in the 79 GHz frequency band is characterized by having a wider usable frequency bandwidth than other millimeter/quasi-millimeter wave radars, such as radars in the 24 GHz, 60 GHz, and 76 GHz frequency bands, for example. The following describes such an embodiment as an example.

The radar scheme of the FMCW radar used in the present disclosure may include a fast-chirp modulation (FCM) scheme that transmits chirp signals on a shorter period than usual. The signal that the electronic device 1 generates is not limited to a signal of the FMCW scheme. The signal that the electronic device 1 generates may also be a signal of any of various schemes other than the FMCW scheme. A transmission signal sequence stored in any storage unit may be different depending on these various schemes. For example, in the case of a radar signal of the FMCW scheme described above, a signal of increasing frequency and a signal of decreasing frequency at each time sample may be used. Known technologies can be applied, as appropriate, for the various schemes described above, and thus a more detailed description is omitted.

As illustrated in FIG. 2, in an embodiment, the electronic device 1 is provided with a signal processing unit 10. The signal processing unit 10 may be provided with a signal generation processing unit 11 and a received signal processing unit 12. The signal generation processing unit 11 and the received signal processing unit 12 will be further described later.

In an embodiment, the electronic device 1 is provided with a transmission DAC 21, a transmission circuit 22, a millimeter-wave transmission circuit 23, and the transmitting antenna array 24 as the transmission unit. In an embodiment, the electronic device 1 is provided with the receiving antenna array 31, a mixer 32, a reception circuit 33, and a reception ADC 34 as the reception unit. In an embodiment, the electronic device 1 need not include at least one of the functional units illustrated in FIG. 2, and may also include a functional unit other than the functional units illustrated in FIG. 2. The electronic device 1 illustrated in FIG. 2 may be formed using a circuit configured in basically the same and/or similar way as a common radar using electromagnetic waves in the millimeter-wave band or the like. On the other hand, in the electronic device 1 according to an embodiment, the signal processing by the signal processing unit 10 may include processing different from the processing performed by a common radar of the related art.

In an embodiment, the signal processing unit 10 provided in the electronic device 1 can control operations by the electronic device 1 as a whole, including control of each of the functional units that make up the electronic device 1. In particular, the signal processing unit 10 performs various processing with respect to signals handled by the electronic device 1. To provide control and processing power for executing various functions, the signal processing unit 10 may include at least one processor, such as a central processing unit (CPU) or a digital signal processor (DSP). The signal processing unit 10 may be realized entirely with a single processor, with several processors, or with respectively discrete processors. The processor may be achieved as a single integrated circuit. An integrated circuit is also referred to as an IC. The processor may be achieved as a plurality of communicatively connected integrated circuits and discrete circuits. The processor may be achieved on the basis of any of various other known technologies. In an embodiment, the signal processing unit 10 may be configured as a CPU (hardware) and a program (software) executed by the CPU, for example. The signal processing unit 10 may include a storage unit (memory) required for operations by the signal processing unit 10.

The signal generation processing unit 11 of the signal processing unit 10 generates a signal to be transmitted from the electronic device 1. In the electronic device 1 according to an embodiment, the signal generation processing unit 11 may generate a transmission signal such as a chirp signal (transmission chirp signal). In particular, the signal generation processing unit 11 may generate signals (linear chirp signals) whose frequency varies periodically and linearly. For example, the signal generation processing unit 11 may generate chirp signals whose frequency increases periodically and linearly from 77 GHz to 81 GHz over time. As another example, the signal generation processing unit 11 may generate periodically repeating signals whose frequency linearly increases (up-chirp) from 77 GHz to 81 GHz and then decreases (down-chirp) over time. The signal that the signal generation processing unit 11 generates may also be preset in the signal processing unit 10, for example. The signal that the signal generation processing unit 11 generates may also be stored in advance in any storage unit or the like in the signal processing unit 10, for example. Since chirp signals used in technical fields such as radar are already known, a more detailed description is simplified or omitted, as appropriate. The signal generated by the signal generation processing unit 11 is supplied to the transmission DAC 21. For this reason, the signal generation processing unit 11 may be connected to the transmission DAC 21.

The transmission DAC (digital-to-analog converter) 21 functions to convert a digital signal supplied from the signal generation processing unit 11 to an analog signal. The transmission DAC 21 may include a common digital-to-analog converter. The signal converted to analog by the transmission DAC 21 is supplied to the transmission circuit 22. For this reason, the transmission DAC 21 may be connected to the transmission circuit 22.

The transmission circuit 22 functions to convert the signal converted to analog by the transmission DAC 21 to an intermediate frequency (IF) band. The transmission circuit 22 may include a common IF-band transmission circuit. The signal processed by the transmission circuit 22 is supplied to the millimeter-wave transmission circuit 23. For this reason, the transmission circuit 22 may be connected to the millimeter-wave transmission circuit 23.

The millimeter-wave transmission circuit 23 functions to transmit the signal processed by the transmission circuit 22 as a millimeter wave (RF wave). The millimeter-wave transmission circuit 23 may include a common millimeter-wave transmission circuit. The signal processed by the millimeter-wave transmission circuit 23 is supplied to the transmitting antenna array 24. For this reason, the millimeter-wave transmission circuit 23 may be connected to the transmitting antenna array 24. The signal processed by the millimeter-wave transmission circuit 23 is also supplied to the mixer 32. For this reason, the millimeter-wave transmission circuit 23 may also be connected to the mixer 32.

The transmitting antenna array 24 is a plurality of transmitting antennas arranged into an array. In FIG. 2, the configuration of the transmitting antenna array 24 is illustrated in a simplified manner. The transmitting antenna array 24 transmits a signal processed by the millimeter-wave transmission circuit 23 to the outside of the electronic device 1. The transmitting antenna array 24 may include a transmitting antenna array used in a common millimeter-wave radar.

In this way, in an embodiment, the electronic device 1 is provided with a transmitting antenna (transmitting antenna array 24) and can transmit a transmission signal (transmission chirp signal, for example) as a transmission wave from the transmitting antenna array 24.

As an example, suppose the case in which an object (for example, a human) such as the occupant 200 is present in the surroundings of the electronic device 1, as illustrated in FIG. 2. In this case, at least a portion of the transmission wave transmitted from the transmitting antenna array 24 is reflected by an object such as the occupant 200. The at least a portion of the transmission wave transmitted from the transmitting antenna array 24 that is reflected by an object such as the occupant 200 may be reflected toward the receiving antenna array 31.

The receiving antenna array 31 receives a reflected wave. The reflected wave may refer to at least a portion of a transmission wave transmitted from the transmitting antenna array 24 that is reflected by an object such as the occupant 200.

The receiving antenna array 31 is a plurality of receiving antennas arranged into an array. In FIG. 2, the configuration of the receiving antenna array 31 is illustrated in a simplified manner. The receiving antenna array 31 receives a reflected wave resulting from a transmission wave transmitted from the transmitting antenna array 24 being reflected. The receiving antenna array 31 may include a receiving antenna array used in a common millimeter-wave radar. The receiving antenna array 31 supplies a received signal received as a reflected wave to the mixer 32. For this reason, the receiving antenna array 31 may be connected to the mixer 32.

The mixer 32 converts a signal (transmission signal) processed by the millimeter-wave transmission circuit 23 and a received signal received by the receiving antenna array 31 to an intermediate frequency (IF) band. The mixer 32 may include a mixer used in a common millimeter-wave radar. The mixer 32 supplies a signal generated as a synthesized result to the reception circuit 33. For this reason, the mixer 32 may be connected to the reception circuit 33.

The reception circuit 33 functions to perform analog processing on a signal converted to an IF band by the mixer 32. The reception circuit 33 may include a common reception circuit that performs conversion to an IF band. The signal processed by the reception circuit 33 is supplied to the reception ADC 34. For this reason, the reception circuit 33 may be connected to the reception ADC 34.

The reception ADC (analog-to-digital converter) 34 functions to convert an analog signal supplied from the reception circuit 33 to a digital signal. The reception ADC 34 may include a common analog-to-digital converter. The signal converted to digital by the reception ADC 34 is supplied to the received signal processing unit 12 of the signal processing unit 10. For this reason, the reception ADC 34 may be connected to the signal processing unit 10.

The received signal processing unit 12 of the signal processing unit 10 functions to perform various processing on a digital signal supplied from the reception ADC 34. For example, the received signal processing unit 12 calculates the distance from the electronic device 1 to an object such as the occupant 200 on the basis of a digital signal supplied from the reception ADC 34 (distance measurement). The received signal processing unit 12 also calculates the relative velocity of an object such as the occupant 200 relative to the electronic device 1 on the basis of a digital signal supplied from the reception ADC 34 (velocity measurement). The received signal processing unit 12 further calculates the bearing angle of an object such as the occupant 200 as seen from the electronic device 1 on the basis of a digital signal supplied from the reception ADC 34 (angle measurement or angle-of-arrival estimation). Specifically, I/Q converted data may be inputted into the received signal processing unit 12. By accepting the input of such data, the received signal processing unit 12 performs a fast Fourier transform (two-dimensional fast Fourier transform (2D-FFT)) process in each of the distance (range) and velocity directions. The received signal processing unit 12 then performs false alarm suppression and fixed probability conversion through the removal of noise points by executing processing such as constant false alarm rate (CFAR). The received signal processing unit 12 performs angle-of-arrival estimation for points that satisfy the CFAR criterion to obtain the position of an object such as the occupant 200. In an embodiment, the received signal processing unit 12 need not perform CFAR processing to perform angle-of-arrival estimation. The information generated as a result of the distance measurement, velocity measurement, and angle measurement (angle-of-arrival estimation) by the received signal processing unit 12 may be supplied to a communication interface 50. Various information outputted as a result of being processed by the received signal processing unit 12 may also be supplied to the communication interface 50. For this reason, the signal processing unit 10 may be connected to the communication interface 50. Various information resulting from arithmetic processing, computational processing, and/or the like by the signal processing unit 10 may also be supplied to another functional unit other than the communication interface 50.

The communication interface 50 is configured to include an interface that outputs information supplied from the signal processing unit 10 to, for example, an external device 60. The communication interface 50 may output information on at least one of the position, velocity, angle, and/or the like of an object such as the occupant 200 as a controller area network (CAN) or other signal, for example, to the external device 60 or the like. For example, information on at least one of the position, velocity, and angle of an object such as the occupant 200 may be supplied to the external device 60 or the like via the communication interface 50. For this reason, the communication interface 50 may be connected to the external device 60 or the like. In an embodiment, various information resulting from arithmetic processing, computational processing, and/or the like by the signal processing unit 10 may also be supplied to, for example, the external device 60 via the communication interface 50.

As illustrated in FIG. 2, in an embodiment, the electronic device 1 may be connected to the external device 60 in a wired or wireless manner via the communication interface 50. In an embodiment, the external device 60 may be configured to include a computer of any kind, a control device of any kind, and/or the like. In an embodiment, the electronic device 1 may be configured to include the external device 60. The external device 60 may be provided with a display unit such as a display that displays images and/or video of any kind. The external device 60 may also be provided with a sound output unit such as a speaker that outputs sound and/or speech of any kind. The external device 60 may also be provided with a tactile sensation presentation unit that presents a prescribed tactile sensation, such as vibration or click-to-click, to the user of the electronic device 1. By being configured in this way, the external device 60 can convey a processing result from the signal processing unit 10 to the user or the like of the electronic device 1 as visual information, auditory information, and/or tactile information, for example.

FIG. 3 is a diagram for describing an example of chirp signals generated by the signal generation processing unit 11 of the signal processing unit 10.

FIG. 3 illustrates the temporal structure of one frame in the case of using the fast-chirp modulation (FCM) scheme. FIG. 3 illustrates an example of a received signal of the FCM scheme. FCM is a scheme in which chirp signals illustrated as c1, c2, c3, c4, ..., cn in FIG. 3 are repeated at short intervals (for example, equal to or greater than the round-trip time of electromagnetic waves between the radar and the target as calculated from the maximum ranging distance). In FCM, the transmission and reception processing is often divided into subframe units as illustrated in FIG. 3 for convenient signal processing of a received signal.

In FIG. 3, the horizontal axis represents elapsed time, and the vertical axis represents frequency. In the example illustrated in FIG. 3, the signal generation processing unit 11 generates linear chirp signals whose frequency varies periodically and linearly. In FIG. 3, the chirp signals are indicated as c1, c2, c3, c4, ..., cn. As illustrated in FIG. 3, in each of the chirp signals, the frequency increases linearly over time.

In the example illustrated in FIG. 3, several chirp signals such as c1, c2, c3, c4, ..., cn are included as a single subframe. That is, subframe 1, subframe 2, and so on illustrated in FIG. 3 are each configured to include several chirp signals such as c1, c2, c3, c4, ..., cn. In the example illustrated in FIG. 3, several subframes such as subframe 1, subframe 2, ..., subframe N are included as a single frame (1 frame). That is, the 1 frame illustrated in FIG. 3 is configured to include N subframes. The 1 frame illustrated in FIG. 3 may be frame 1, followed by frame 2, frame 3, and so on. These frames are each configured to include N subframes, in the same and/or similar manner as frame 1. A frame interval of given length may also be included between frames. The single frame illustrated in FIG. 3 may be around 30-50 milliseconds long, for example.

In the electronic device 1 according to an embodiment, the signal generation processing unit 11 may generate a transmission signal as any number of frames. In FIG. 3, some of the chirp signals are omitted from illustration. The relationship between the time and the frequency of a transmission signal generated by the signal generation processing unit 11 in this way may be stored in a storage unit or the like of the signal processing unit 10.

In this way, in an embodiment, the electronic device 1 may transmit a transmission signal formed from subframes including a plurality of chirp signals. In an embodiment, the electronic device 1 may transmit a transmission signal formed from frames including a given number of subframes.

The following describes the electronic device 1 as transmitting a transmission signal with a frame structure as illustrated in FIG. 3. However, the frame structure as illustrated in FIG. 3 is an example, and the chirp signals included in one subframes may be of any kind, for example. That is, in an embodiment, the signal generation processing unit 11 may generate subframes including any number of (for example, any plurality of) chirp signals. The subframe structure illustrated in FIG. 3 is also an example, and the subframes included in one frame may be of any kind, for example. That is, in an embodiment, the signal generation processing unit 11 may generate frames including any number of (for example, any plurality of) subframes. The signal generation processing unit 11 may generate signals of different frequency. The signal generation processing unit 11 may generate a plurality of discrete signals each having a different frequency f.

FIG. 4 illustrates another form of a portion of the subframes illustrated in FIG. 3. FIG. 4 is an illustration of samples of a received signal obtained by receiving the transmission signal illustrated in FIG. 3, as a result of performing processing, namely the 2D-FFT, in the received signal processing unit 12 (FIG. 2) of the signal processing unit 10.

As illustrated in FIG. 4, each of chirp signals c1, c2, c3, c4, ..., cn is stored in each of subframes such as subframe 1, ..., subframe N. In FIG. 4, each of the chirp signals c1, c2, c3, c4, ..., cn is formed from samples, which are indicated by the horizontally arrayed grid squares. The received signal illustrated in FIG. 4 is subjected to 2D-FFT, CFAR, integration signal processing on each subframe, and/or the like by the received signal processing unit 12 illustrated in FIG. 2.

FIG. 5 illustrates an example of a point group in the range-Doppler (distance-velocity) plane calculated as a result of performing 2D-FFT, CFAR, and integration signal processing on each subframe in the received signal processing unit 12 illustrated in FIG. 2.

In FIG. 5, the horizontal direction represents range (distance), and the vertical direction represents velocity. The shaded grid square s1 illustrated in FIG. 5 illustrates a point group indicating a signal exceeding CFAR threshold processing. The non-shaded grid square s2 illustrated in FIG. 5 illustrates a bin (2D-FFT sample) with no point group, which did not exceed the CFAR threshold. Direction estimation is used to calculate the bearing, from the radar, of the calculated point group in the range-Doppler plane illustrated in FIG. 5, and the position and velocity in the two-dimensional plane are calculated as a point group indicating an object such as the occupant 200. Direction estimation may be calculated by a beamformer and/or a subspace method. Typical subspace method algorithms include MUltiple SIgnal Classification (MUSIC) and estimation of signal parameters via rotational invariant techniques (ESPRIT).

FIG. 6 illustrates an example of the result of the transformation of point group coordinates from the range-Doppler plane illustrated in FIG. 5 to the XY plane by the received signal processing unit 12 after performing direction estimation. As illustrated in FIG. 6, the received signal processing unit 12 can plot a point group PG in the XY plane. The point group PG contains points P (with coordinates (x, y)). Each of the points P has an angle $\theta$ and a radial velocity Vr in polar coordinates.

The received signal processing unit 12 detects an object present in the range where a transmission wave T was transmitted, on the basis of at least one of the 2D-FFT or angle estimation results. The received signal processing unit 12 may perform object detection by performing, for example, clustering processing on the basis of respectively estimated information on distance, information on velocity, and angle information. Known algorithms used in clustering data include density-based spatial clustering of applications with noise (DBSCAN), for example. DBSCAN is an algorithm that performs density-based clustering. In the clustering processing, the average power of the points making up a detected object may be calculated, for example. The information on distance, information on velocity, angle information, and information on power pertaining to an object detected in the received signal processing unit 12 may be supplied to the external device 60 or the like via the communication interface 50, for example.

As above, the electronic device 1 may be provided with a transmitting antenna (transmitting antenna array 24), a receiving antenna (receiving antenna array 31), and the signal processing unit 10. The transmitting antenna array 24 transmits a transmission wave T. The receiving antenna array 31 receives a reflected wave R resulting from the transmission wave T being reflected. The signal processing unit 10 detects an object (an object such as the occupant 200, for example) that reflects the transmission wave T, on the basis of the transmission signal transmitted as the transmission wave T and the received signal received as the reflected wave R.

The following further describes direction estimation of an arriving wave (angle-of-arrival estimation) by an antenna array of the electronic device 1 according to an embodiment.

FIG. 7 is a diagram for describing the configuration of the receiving antenna array 31 and the principle of direction estimation of an arriving wave by the receiving antenna array 31 of the electronic device 1 according to an embodiment. FIG. 7 illustrates an example of the reception of radio waves by the receiving antenna array 31.

As illustrated in FIG. 7, the receiving antenna array 31 may be a linear arrangement of sensors such as receiving antennas. As illustrated in FIG. 7, in an embodiment, the receiving antenna array 31 may be configured to include a plurality of receiving antennas in a linear array. In FIG. 7, the plurality of antennas $x_1$, $x_2$, $x_3$, ..., $x_M$ that make up the receiving antenna array 31 are illustrated by small circles. FIG. 7 schematically illustrates the arrangement of the plurality of antennas that make up the receiving antenna array 31. The actual shapes of the plurality of antennas that make up the receiving antenna array 31 may be shapes different from small circles, such as patch antennas, for example. The receiving antenna array 31 may include any plurality of antennas. As illustrated in FIG. 7, the plurality of antennas that make up the receiving antenna array 31 are spaced apart from one another by an array pitch d. This type of sensor array, in which sensors (such as antennas, ultrasonic transducers, and microphones) corresponding to various physical waves are disposed in an array, is also referred to as a uniform linear array (ULA). As illustrated in FIG. 7, the physical waves (such as electromagnetic waves and sonic waves) arrive from various directions, such as $\theta_1$ and $\theta_2$, for example. Herein, $\theta_1$ and $\theta_2$ may refer to the (estimated) angle of arrival described above. In this way, a sensor array like the receiving antenna array 31 can estimate the direction of arrival (angle of arrival) by utilizing the phase difference that occurs in measurement values between sensors according to the direction of arrival of a physical wave. This type of technique for estimating the direction of arrival of a wave is also referred to as arriving angle estimation, angle-of-arrival estimation, or direction-of-arrival (DoA) estimation.

In the electronic device 1 according to an embodiment, at least one of the transmitting antenna array 24 or the receiving antenna array 31 may have a plurality of antennas in a linear arrangement. This allows for appropriate narrowing of directivity in the transmission and reception of radio waves in a millimeter-wave radar, for example. When transmitting a transmission wave, the direction of the transmission beam is often controlled by a beamformer. On the other hand, when receiving a reflected wave, the direction of arrival of the reflected wave is more often estimated by subspace methods (such as MUSIC and ESPRIT described above) than by a beamformer. With beamformers and subspace methods, for electromagnetic waves arriving from various directions in the ULA as illustrated in FIG. 7, a phase difference occurs in measurement values between sensors depending on the direction of arrival. Accordingly, this phase difference can be utilized to estimate the direction of arrival of a reflected wave.

The following further describes angle estimation in two directions of an arriving wave by an antenna array of the electronic device 1 according to an embodiment.

FIG. 8 illustrates an example arrangement of antennas for estimating the direction of arrival with respect to two orthogonal angles.

As illustrated in FIG. 8, in the electronic device 1 according to an embodiment, the transmitting antenna array 24 and/or receiving antenna array 31 may be configured to include an array of a plurality of patch antenna units.

In the transmitting antenna array 24 illustrated in FIG. 8, one patch antenna unit may be configured to include a plurality of elements electrically connected in the direction of direction 1 illustrated in the diagram. In each of the patch antenna units, the plurality of elements may be electrically connected by wiring such as striplines on a board, for example. In each of the patch antenna units, the plurality of elements may be spaced apart from one another by a pitch $d_{1,t}$ shorter than half the wavelength $\lambda$ of the transmission wave. In FIG. 8, each of the patch antenna units may have any number of two or more elements electrically connected.

As illustrated in FIG. 8, the transmitting antenna array 24 may include a plurality of patch antenna units arrayed in the direction of direction 2 illustrated in the diagram. The patch antenna units may be spaced apart from one another by a pitch $d_{2,t}$ shorter than half the wavelength $\lambda$ of the transmission wave. In an embodiment, the transmitting antenna array 24 may include any number of two or more patch antenna units.

As illustrated in FIG. 8, in an embodiment, the receiving antenna array 31 may be a variation of the arrangement of the plurality of elements in the transmitting antenna array 24. That is, in the receiving antenna array 31 illustrated in FIG. 8, one patch antenna unit may be configured to include a plurality of elements electrically connected in the direction of direction 2 illustrated in the diagram. In each of the patch antenna units, the plurality of elements may be electrically connected by wiring such as striplines on a board, for example. In each of the patch antenna units, the plurality of elements may be spaced apart from one another by a pitch $d_{2,s}$ shorter than half the wavelength $\lambda$ of the transmission wave. In FIG. 8, each of the patch antenna units may have any number of two or more elements electrically connected.

As illustrated in FIG. 8, the receiving antenna array 31 may include a plurality of patch antenna units arrayed in the direction of direction 1 illustrated in the diagram. The patch antenna units may be spaced apart from one another by a pitch $d_{1,s}$ shorter than half the wavelength $\lambda$ of the transmission wave. In an embodiment, the receiving antenna array 31 may include any number of two or more patch antenna units.

The elements included in the transmitting antenna array 24 and the receiving antenna array 31 may all be arranged in the same plane (on the surface layer of the same board, for example). The transmitting antenna array 24 and the receiving antenna array 31 may also be arranged in proximity to each other (monostatic). Direction 1 and direction 2 illustrated in FIG. 8 may be geometrically orthogonal.

With the transmitting antenna array 24 and the receiving antenna array 31 as illustrated in FIG. 8, the directivity of each of the transmitting antenna and the receiving antenna can be narrowed appropriately. By using the transmitting antenna array 24 as illustrated in FIG. 8 to control the direction in which to transmit each transmission wave at each timing for transmitting a transmission wave (transmission signal), a beamformer can be realized for the direction of direction 2 illustrated in FIG. 8. By using the receiving antenna array 31 as illustrated in FIG. 8, estimation of the direction of arrival of the reflected wave can be realized for the direction of direction 1 illustrated in FIG. 8. This enables estimation of the direction of arrival of a reflected wave with respect to two substantially orthogonal angles. Accordingly, a point group indicating an object such as the occupant 200 can be acquired three-dimensionally.

The following describes a technique by which the electronic device 1 according to an embodiment detects the presence and the presence position of an occupant, including the driver, in an interior space of an automobile.

FIG. 9 illustrates an example of the electronic device 1 according to an embodiment installed inside an automobile. FIG. 10 is a diagram illustrating an example of the position where the electronic device 1 according to an embodiment is installed inside an automobile and the positions of seats disposed inside the automobile. In an embodiment, the electronic device 1 may be installed at a position as illustrated in FIGs. 9 and 10, for example. In FIGs. 9 and 10, the automobile is assumed to be a right-hand drive vehicle, or in other words a vehicle with the steering wheel installed on the right side of the direction of travel, which is prevalent in Japan. For example, the driver's seat may be located at the position P1 illustrated in FIG. 10. The passenger seat may be located at the position P2 illustrated in FIG. 10. The driver-side rear seat may be located at the position P3 illustrated in FIG. 10. The passenger-side rear seat may be located at the position P4 illustrated in FIG. 10. On the other hand, in an embodiment, the electronic device 1 may also be installed inside a left-hand drive vehicle. In an embodiment, the automobile in which the electronic device 1 is installed is not limited to what is illustrated in FIG. 9 or 10, and may be any of various types of automobiles. For example, in an embodiment, the automobile in which the electronic device 1 is installed may also have three seats as rear seats. In an embodiment, the automobile in which the electronic device 1 is installed may also have three or more rows of seats.

As illustrated in FIGs. 9 and 10, the electronic device 1 may be installed close to a driver seated in the driver's seat, for example. As illustrated in FIG. 9, the electronic device 1 may be mounted on, or in the vicinity of, an automotive sun visor, for example, or may be mounted on the ceiling of the automobile (the upper part in the cabin), for example. Patent Literature 1 mentioned earlier teaches that a transmitter/receiver is located closest to the driver's seat from among the seats in the cabin. However, in an embodiment, the electronic device 1 need not be located closest to the driver's seat from among the seats in the cabin.

On the other hand, in an embodiment, the electronic device 1 may be installed at a position such that the distances to the people respectively seated in each of the seats are different from each other. In other words, in the example illustrated in FIG. 10, the electronic device 1 may be located such that the distances from the electronic device 1 to the people respectively seated at the positions P1 to P4 are different from each other. For example, let $\alpha 1$ be the distance from the electronic device 1 to the human seated in the seat at the position P1. Let $\alpha 2$ be the distance from the electronic device 1 to the human seated in the seat at the position P2. Let $\alpha 3$ be the distance from the electronic device 1 to the human seated in the seat at the position P3. Let $\alpha 4$ be the distance from the electronic device 1 to the human seated in the seat at the position P4. In this case, the electronic device 1 may be located such that $\alpha 1$, $\alpha 2$, $\alpha 3$, and $\alpha 4$ are mutually different distances (lengths).

In an embodiment, the electronic device 1 may also be located such that the angles from the electronic device 1 to each of the seats are as different as possible. In other words, in an embodiment, the electronic device 1 may be located such that the differences between each of the angles from the electronic device 1 to each of the seats are as large as possible. Such an arrangement of the electronic device 1 allows for a relatively broad view of the body surfaces of the people seated in each of the seats from the electronic device 1. This facilitates detection of the people seated in each of the seats by the electronic device 1 from the standpoint of S/N.

In an embodiment, the electronic device 1 may be pre-installed inside an automobile. In other words, in an embodiment, the electronic device 1 may be shipped in a state of being pre-installed inside an automobile. In an embodiment, the electronic device 1 may also be installed later inside an automobile. In other words, in an embodiment, the electronic device 1 may be shipped to be retrofitted inside an automobile.

As described above, in an embodiment, the electronic device 1 may be configured to include a millimeter-wave radar of the FMCW scheme. In an embodiment, the electronic device 1 is installed in the cabin of an automobile, for example, thereby enabling the detection of an occupant seated in the cabin by a millimeter-wave radar of the FMCW scheme. The surface of the human body is constantly undergoing microvibrations due to body movement, respiration, and/or heartbeat. Therefore, in an embodiment, the electronic device 1 can distinguish a human body from a stationary object by detecting movement caused by vibrations of the human body with a millimeter-wave radar sensor.

On the other hand, if detection is performed using a millimeter-wave sensor in a space that is at least partially enclosed, such as in the cabin of an automobile, for example, the problem known as multipath arises. Multipath is a phenomenon caused by multiple reflections and is known to be particularly likely to occur in enclosed spaces, such as in the cabin of an automobile, for example. Radio waves transmitted from a transmitting antenna not only travel directly to and from a target object, but some are also received by a receiving antenna after being reflected one or more times by surrounding objects. If object detection is performed on the basis of the signal of the multipath component of a radio wave that traveled along a superfluous path, the object is detected as though existing at a greater distance than the position where the object actually exists. Signal processing performed on the basis of a received signal received in this way may act as a factor that leads to false detection or impedes correct detection. For example, if a radar sensor is used to detect a plurality of occupants seated in a cabin, multipath may occur due to an occupant seated in a seat closer to the radar sensor. This may reduce the accuracy of detecting an occupant seated in a seat farther away from the radar sensor. For example, if processing is performed to derive a point group with high-intensity 2D Fourier transform results by constant false alarm rate (CFAR) processing, the presence of an occupant seated in a seat close to the radar sensor may cause false detection of an occupant seated in a seat to the rear.

FIG. 11 is a diagram illustrating an example of a result in which an occupant is detected by a radar sensor in the cabin of an automobile. FIG. 11 may, for example, illustrate the signal intensity obtained as a result of receiving a radio wave transmitted by the electronic device 1 in the situation (inside an automobile) illustrated in FIGs. 9 and 10. FIG. 11 illustrates an example of the result of executing a two-dimensional fast Fourier transform (2D-FFT) process on a received signal detected in a situation in which only one occupant is present in the cabin of an automobile. FIG. 11 illustrates the result of executing a Fourier transform process twice on a signal received by the electronic device 1. The first Fourier transform can be used to derive a distance component from the received signal. The second Fourier transform can be used to derive a velocity component. In the graph illustrated in FIG. 11, the horizontal axis represents velocity and the vertical axis represents distance (range). In the graph illustrated in FIG. 11, a color closer to white represents a region of higher signal intensity, while a color closer to black represents a region of lower signal intensity. In the graph illustrated in FIG. 11, the region closer to black that occupies most portions corresponds to the intensity of noise in the signal detected by the electronic device 1. The region labeled "detection of direct waves" (the region corresponding to short distances) in FIG. 11 represents the region where radio waves travel directly to and from the electronic device 1 and the occupant. As illustrated in FIG. 11, the region labeled "detection of direct waves" is detected as a component with a velocity. This region is due to changes in body surface as the occupant breathes. In this way, the occupant is detected as a component with a velocity, which is due to changes in body surface associated with respiration. As illustrated in FIG. 11, a signal of some intensity is also detected in the region (the region labeled "effect due to multipath") a short distance away from the region labeled "detection of direct waves". This region represents a region where radio waves not traveling directly to and from the electronic device 1 and the occupant are detected, or in other words a region caused by the effect due to multipath. The component of the signal detected in the region of zero or near-zero velocity at the left edge of the graph illustrated in FIG. 11 is mainly due to reflections from stationary objects present in the cabin. As illustrated in FIG. 11, if detection is performed using a millimeter-wave sensor in the cabin of an automobile, the multipath problem arises. Therefore, if a signal detected using a millimeter-wave sensor in the cabin of an automobile is used as-is, the result may lead to false detection or impede correct detection. However, according to an embodiment, the electronic device 1 can distinguish the seat where an occupant is seated with relatively high accuracy, even in an environment where the effects of multipath as described above occur.

The distance resolution of a radar sensor of the FMCW scheme depends on the occupied bandwidth. Typically, detection using a millimeter-wave sensor has a distance resolution of several centimeters or more. Consequently, the resolution of detection using a millimeter-wave sensor is hardly sufficient to distinguish between human bodies seated in seats in a relatively confined cabin space such as inside an automobile. The distance between an occupant seated in a seat and a radar sensor may also depend on how the occupant is seated and/or the body type of the occupant. Therefore, depending on the situation in which occupants are seated in respective seats, the resolution of detection by the millimeter-wave sensor may be insufficient. Non-Patent Literature 1 mentioned earlier teaches that an occupant inside an automobile is detected using machine learning to process a detection signal from a millimeter-wave radar. The radar scheme adopted by Non-Patent Literature 1 has relatively low angular resolution and thus is limited to recognizing the row in which an occupant is seated in a cabin, and does not further distinguish the seat in which the occupant is seated within a row of the cabin. However, according to an embodiment, the electronic device 1 can distinguish the seat where an occupant is seated with relatively high accuracy, even when using a sensor with less distance resolution.

In an embodiment, the electronic device 1 records results of detection by a millimeter-wave radar sensor for all patterns of occupants seated in seats inside an automobile, and uses the results as supervisory data to distinguish seated occupants. With such an approach, in an embodiment, the electronic device 1 can correctly distinguish the location of an occupant seated in a cabin, even in an environment where multipath occurs and even when using a sensor with less distance resolution. Consequently, according to an embodiment, the electronic device 1 can distinguish the seat in which a seated occupant is present in a cabin by ascertaining in advance the distances from the electronic device 1 to each of the seats.

According to an embodiment, the electronic device 1 can detect occupants seated in the seats located at the positions P1 to P4 in the cabin of an automobile as illustrated in FIG. 10, for example. According to an embodiment, the electronic device 1 can determine whether or not an occupant is seated in at least one of the seats located at the positions P1 to P4 in the cabin of an automobile as illustrated in FIG. 10, for example. According to an embodiment, the electronic device 1 can also determine whether or not an occupant is seated in each of the seats located at the positions P1 to P4 in the cabin of an automobile as illustrated in FIG. 10, for example. Consequently, according to an embodiment, the electronic device 1 can transmit and receive radio waves to detect with good accuracy the presence and the presence position of a human or the like in an at least a partially enclosed space such as the cabin of an automobile, for example.

Operations by the electronic device 1 according to an embodiment may include the following two phases, for example.

(1) Generate model by machine learning based on supervisory data
(2) Distinguish presence position of occupant on the basis of generated model

The operations in (1) are for performing machine learning on the basis of supervisory data acquired in advance before actually distinguishing the seating of an occupant by the electronic device 1 according to an embodiment. The operations in (2) are for actually distinguishing the seating of an occupant by the electronic device 1 according to an embodiment on the basis of the result of the machine learning performed in (1).

FIG. 12 is a flowchart for describing operations by the electronic device 1 according to an embodiment. FIG. 12 may illustrate "(1) Generate model by machine learning based on supervisory data" above. In other words, the operations illustrated in FIG. 12 may be for performing machine learning on the basis of supervisory data acquired in advance before actually distinguishing the seating of an occupant by the electronic device 1 according to an embodiment.

The following assumes that at the time when the operations illustrated in FIG. 12 start, the electronic device 1 has been installed in the cabin of an automobile as illustrated in FIG. 10, for example. The following assumes that at the time when the operations illustrated in FIG. 12 start, any number of occupants from 0 persons up to, for example, 4 persons are seated in seats in the cabin of the automobile as illustrated in FIG. 10, for example, in which the electronic device 1 is installed. If there are between one and three occupants, for example, each of the occupants may be seated in any of the seats at the positions P1 to P4 in the cabin of the automobile as illustrated in FIG. 10, for example. In the following, a description may be simplified or omitted for general operations and processes performed when a known millimeter-wave radar transmits and receives radio waves.

When the operations illustrated in FIG. 12 start, the signal processing unit 10 of the electronic device 1 according to an embodiment carries out control to transmit a transmission wave from a transmitting antenna 25 of the electronic device 1 (step S11). The transmission signal transmitted in step S11 may be a chirp signal as illustrated in FIG. 3, for example.

Once the transmission wave is transmitted in step S11, the signal processing unit 10 carries out control to receive, from a receiving antenna 31 of the electronic device 1, a reflected wave resulting from the transmission wave being reflected by an object (step S12). The received signal received in step S12 may be based on a reflected wave resulting from the transmission wave being reflected by the occupant seated in the cabin of the automobile, for example. The received signal received in step S12 may be based on a reflected wave resulting from the transmission wave being reflected by another object, such as a stationary object, in the cabin of the automobile, for example.

Once the reflected wave is received in step S12, the signal processing unit 10 acquires a received signal (ADC data) that has been converted from analog to digital by the reception ADC 34 (step S13).

Upon acquiring ADC data in step S13, the signal processing unit 10 performs a distance FFT process and a velocity FFT process (2D-FFT process) on the acquired ADC data (a beat signal based on the transmission wave and the reflected wave) (step S14). In step S14, the signal processing unit 10 may execute a Fourier transform process twice on the acquired ADC data. The first Fourier transform is used to derive a distance component from the received signal. The second Fourier transform is used to derive a velocity component. The result of the process executed in step S14 can be expressed like the graph illustrated in FIG. 11, for example. As described above, FIG. 11 illustrates an example of the result of executing a 2D-FFT process on a received signal detected by a radar sensor in a situation in which only one occupant is present in the cabin of an automobile.

The signal processing unit 10 may perform angle-of-arrival estimation on the basis of the result of performing the 2D-FFT process in step S14 (step S15). In step S15, the signal processing unit 10 may use the ESPRIT method, for example, as the technique for angle-of-arrival estimation. The result of the 2D-FFT process executed in step S14 can be illustrated like the left side of FIG. 13, for example. The diagram illustrated on the left side of FIG. 13 conceptually illustrates the results of executing the 2D-FFT process on a signal received from multiple channels (reception channels). In the diagram illustrated on the left side of FIG. 13, the results of executing the 2D-FFT process on each of signals received by multiple receiving antennas 31 are arranged side by side from left to right. In the diagram illustrated on the left side of FIG. 13, the direction from top to bottom represents distance (range) and the direction from front to back represents velocity. The diagram illustrated on the left side of FIG. 13 may illustrate the results when there are four reception channels, that is, the results of executing the 2D-FFT process on each of signals received by four receiving antennas 31. The four receiving antennas 31 may include a first receiving antenna 31a, a second receiving antenna 31b, a third receiving antenna 31c, and a fourth receiving antenna 31d, for example. In the electronic device 1 according to an embodiment, the number of receiving antennas 31 is not limited to four and may be any plural number. In this case, in the diagram illustrated on the left side of FIG. 13, the results of executing the 2D-FFT process on each of signals received by the any plural number of receiving antennas 31 may be arranged side by side from left to right.

As illustrated on the left side of FIG. 13, the results of the 2D-FFT process in step S14 can be expressed as a three-dimensional data structure with the three axes of distance, velocity, and reception channel (the signals received from multiple receiving antennas 31). The data arrayed three-dimensionally as illustrated on the left side of FIG. 13 may be referred to as a "three-dimensional array", "three-dimensional data", or the like. In step S15, the signal processing unit 10 estimates the direction of arrival (angle of arrival) of a signal by using the ESPRIT method, for example, on the basis of the amplitudes and the phase differences on the axis in the direction of the multiple reception channels (the axis from left to right). The result of the angle-of-arrival estimation executed in step S15 can be illustrated like the right side of FIG. 13, for example. In other words, when angle-of-arrival estimation is executed in step S15 on the basis of the three-dimensional data structure illustrated on the left side of FIG. 13, a result as illustrated on the right side of FIG. 13 is generated. As illustrated on the right side of FIG. 13, the result of the direction-of-arrival estimation executed in step S15 is a data structure expressing angles with the two coordinates of distance and velocity. In the diagram illustrated on the right side of FIG. 13, the direction from top to bottom represents distance (range) and the direction from front to back represents velocity, as in the case of the diagram illustrated on the left side of FIG. 13. In the data structure illustrated on the right side of FIG. 13, a single value indicating an angle is mapped to each point at coordinates indicating a distance and a velocity generated as a result of the 2D-FFT. In other words, a single value indicating an angle exists inside each of the cubes (blocks) that make up the data structure illustrated on the right side of FIG. 13. The data arrayed two-dimensionally as illustrated on the right side of FIG. 13 may be referred to as a "two-dimensional array", "two-dimensional data", or the like.

Once the angle-of-arrival estimation is executed in step S15, the signal processing unit 10 determines whether or not the process from step S11 to step S15 has been executed a prescribed number of times (step S16). The prescribed number of times to be determined in step S16 may be a number of times that the process from step S11 to step S15 is to be repeated within a prescribed time, for example. More specifically, the prescribed number of times to be determined in step S16 may be a number of times that the process from step S11 to step S15 is to be repeated before acquisition of the data constituting the three-dimensional data to be generated in step S17 described later. Upon determining in step S16 that the process thus far has not been executed the prescribed number of times, the signal processing unit 10 returns to step S11 and repeats the process to step S15.

On the other hand, upon determining in step S16 that the process thus far has been executed the prescribed number of times, the signal processing unit 10 executes the process in step S17. In step S17, the signal processing unit 10 generates three-dimensional data having a time axis as illustrated in FIG. 14, for example, as a result of executing the process from step S11 to step S15 the prescribed number of times. The three-dimensional data illustrated in FIG. 14 is a three-dimensional array of data indicating the results of the angle-of-arrival estimation. In the three-dimensional data illustrated in FIG. 14, the direction from top to bottom represents distance (range) and the direction from front to back represents velocity, as in the case of the diagrams illustrated in FIG. 13. In the three-dimensional data illustrated in FIG. 14, the direction from left to right represents time. In other words, the three-dimensional data illustrated in FIG. 14 may contain [i] pieces of data in the distance (range) direction, [j] pieces of data in the velocity direction, and [t] pieces of data in the time direction. Let $A_{ijt}$ be a point group indicating the results of angle-of-arrival estimation constituting three-dimensional data as illustrated in FIG. 14. $A_{ijt}$ is information indicating the estimated angle of arrival that is i-th in the distance (range) direction, j-th in the velocity direction, and t-th in the time direction. The distance i is assumed to satisfy $0 \leq i \leq I$, the velocity j is assumed to satisfy $0 \leq j \leq J$, and the time t is assumed to satisfy $0 \leq t \leq T$. In step S17, the signal processing unit 10 may generate the three-dimensional data illustrated in FIG. 14 by gathering a plurality of the two-dimensional data illustrated on the right side of FIG. 13 and arranging the data side by side from left to right. The minimum unit of time indicated by the direction from left to right in the three-dimensional data illustrated in FIG. 14 may be the time to acquire ADC data for one iteration of the 2D-FFT process.

The number of pieces of data arranged in each of the directions from top to bottom, left to right, and/or front to back in the three-dimensional data illustrated in FIG. 14 is not limited to the example illustrated in FIG. 14. The number of pieces of data arranged in each of the directions from top to bottom, left to right, and/or front to back in the three-dimensional data illustrated in FIG. 14 may be set appropriately, as necessary. As an example, assume that the time to acquire ADC data necessary for the signal processing unit 10 to perform one iteration of the 2D-FFT process is 64 ms. In this case, the signal processing unit 10 may generate time-series three-dimensional data as illustrated in FIG. 14 in step S17 by performing the process from step S11 to step S15 a total of 240 times. In this case, the temporal length to acquire ADC data is around 0.064 seconds * 240 times, or in other words 15.36 seconds. On the other hand, if acquiring data over a longer time would improve the accuracy of the model to be generated as supervisory data, time-series three-dimensional data as illustrated in FIG. 14 may also be generated on the basis of data over a longer time.

Once the three-dimensional data is generated in step S17, the signal processing unit 10 extracts prescribed features on the basis of the three-dimensional data (step S18). The following describes in greater detail the operations executed by the signal processing unit 10 in step S18.

In step S18, the signal processing unit 10 first chooses the two-dimensional data corresponding to several distance (range) coordinates as illustrated in FIG. 15 from the three-dimensional data as illustrated in FIG. 14. In other words, the signal processing unit 10 chooses n pieces of two-dimensional data as illustrated in FIG. 15 from among the three-dimensional data containing i pieces of data in the distance direction as illustrated in FIG. 14. The n pieces of two-dimensional data illustrated in FIG. 15 each have axes in the velocity and time directions. In other words, each of the n pieces of two-dimensional data illustrated in FIG. 15 may contain m pieces of data in the time direction. The n pieces of two-dimensional data chosen at this point may correspond to the distance (range) from the electronic device 1 to each occupant seated in a seat located inside an automobile. For example, as described above, let $\alpha1$ be the distance from the electronic device 1 illustrated in FIG. 10 to the human seated in the seat at the position P1, and let $\alpha2$ be the distance from the electronic device 1 to the human seated in the seat at the position P2. Let $\alpha3$ be the distance from the electronic device 1 to the human seated in the seat at the position P3, and let $\alpha4$ be the distance from the electronic device 1 to the human seated in the seat at the position P4. In this case, a total of n pieces of two-dimensional data corresponding to the distance (range) of each of the distances $\alpha1$, $\alpha2$, $\alpha3$, and $\alpha4$ may be chosen. For example, if the distance $\alpha1$ from the electronic device 1 to the human seated in the seat at the position P1 is 0.4 m, two-dimensional data corresponding to the distance (range) of 0.4 m may be chosen. If the distance $\alpha2$ from the electronic device 1 to the human seated in the seat at the position P2 is 0.7 m, two-dimensional data corresponding to the distance (range) of 0.7 m may be chosen. If the distance $\alpha3$ from the electronic device 1 to the human seated in the seat at the position P3 is 1.5 m, two-dimensional data corresponding to the distance (range) of 1.5 m may be chosen. If the distance $\alpha4$ from the electronic device 1 to the human seated in the seat at the position P4 is 1.9 m, two-dimensional data corresponding to the distance (range) of 1.9 m may be chosen. The two-dimensional data corresponding to the distance (range) of each of the distances $\alpha1$, $\alpha2$, $\alpha3$, and $\alpha4$ may be chosen one at a time for each distance (range), or may be chosen several at a time for each distance (range). For example, if the distance $\alpha$1 from the electronic device 1 to the human seated in the seat at the position P1 is 0.4 m, two-dimensional data corresponding to the distance (range) of 0.39 m and two-dimensional data corresponding to the distance (range) of 0.41 m or the like may be chosen.

The example given below is for the case where the n pieces of data illustrated in FIG. 15 are 9 in number (n = 9). The breakdown of n = 9 may be as follows, for example. Two pieces of two-dimensional data corresponding to the distance $\alpha$1 from the electronic device 1 to the human seated in the seat at the position P1 are chosen, and two pieces of two-dimensional data corresponding to the distance $\alpha$2 from the electronic device 1 to the human seated in the seat at the position P2 are chosen. A total of five pieces of two-dimensional data corresponding to the distance $\alpha$3 from the electronic device 1 to the human seated in the seat at the position P3 and the distance $\alpha$4 from the electronic device 1 to the human seated in the seat at the position P4 are chosen.

The n pieces of two-dimensional data (see FIG. 15) chosen as described above are pieces of data having the two axes of velocity and time, as illustrated in FIG. 14. In step S18, the signal processing unit 10 extracts prescribed features on the basis of the chosen n pieces of two-dimensional data. The prescribed features to be extracted in step S18 may be at least one from among the following first, second, and third features, for example.

First feature $K_{it}$: average in velocity direction based on chosen n pieces of two-dimensional data
Second feature $S_{it}$: unbiased standard deviation in velocity direction based on chosen n pieces of two-dimensional data
Third feature $T_{it}$: unbiased standard deviation in time direction based on chosen n pieces of two-dimensional data

Extracting all three of the first, second, and third features will result in there being three respective features for a single piece of two-dimensional data, for a total of 3n features. Let m be the number of time-axis coordinates when data with different coordinates on the time axis are treated as discrete pieces of data (see FIGs. 14 and 15). In this way, in step S18, the signal processing unit 10 can generate a table of features containing 3n features arrayed in the horizontal direction and m datasets arrayed in the vertical direction, as illustrated in FIG. 16, for example.

The following further describes a method for extracting each of the first, second, and third features described above.

In step S18, the signal processing unit 10 may extract the first feature $K_{it}$ on the basis of the following expression (1).

[Math. 1]

$$K_{it} = \frac{1}{J}\sum_j A_{ijt} \qquad (1)$$

In expression (1), $A_{ijt}$ represents a point group indicating the results of angle-of-arrival estimation constituting three-dimensional data as illustrated in FIG. 14. In expression (1), i represents the order of data in the distance (range) direction, j represents the order of data in the velocity direction, and t represents the order of data in the time direction.

In step S18, the signal processing unit 10 may extract the second feature $S_{it}$ on the basis of the following expression (2) by using the result of the first feature $K_{it}$.

[Math. 2]

$$S_{it} = \frac{1}{J-1} * \sqrt{\sum_j (A_{ijt} - K_{it})^2} \qquad (2)$$

In step S18, the signal processing unit 10 may extract the third feature $T_{it}$ on the basis of the following expression (3) by using the result of the first feature $K_{it}$.

[Math. 3]

$$T_{it} = \frac{1}{t-t'-1} * \sqrt{\sum_{t-t' \le r' \le t} (K_{ir'} - \frac{1}{t-t'}\sum_{t-t' \le r \le t} K_{ir})^2} \qquad (3)$$

In expression (3), t' may be a constant determined by design, and may be a time of around 2 seconds, for example. In the extraction of the third feature $T_{it}$ expressed in expression (3), the data at time t may be obtained by calculating the unbiased standard deviation over the interval [t-t', t], with t' being a constant, as illustrated in FIG. 17. At time t+1, the unbiased standard deviation may be calculated over the interval [t-t'+1, t+1]. In expression (3), r and r' are variables representing times prior to time t. $T_{it}$ on the left side of expression (3) is a two-dimensional array of distance and time, but generating this array requires looking up not only K at time t but also K at a past time. Consequently, t alone is insufficient as the variable presenting time, and thus the variables r and r' are newly introduced. In the present embodiment, differences in radar outputs for different seat locations can be made explicit by quantifying the change over time in the results of the angle-of-arrival estimation. Expression (3) can be used as an example of a way of quantifying this change over time. Although the present embodiment indicates an example of obtaining the unbiased variance, another method may also be used in regard to the quantified portion. In machine learning, a process for acquiring differences in the value of $T_{it}$ between different seat arrangement patterns may be performed when carrying the calculation in expression (3). If a method other than the unbiased variance is used, a way of quantifying the change over time in the results of the angle-of-arrival estimation may be used.

FIGs. 18 to 20 illustrate examples of plotting the results of extracting the first and third features described above. The results illustrated in FIGs. 18 to 20 are obtained from expressions (1) and (3) described above (the first feature and the third feature) by using the electronic device 1 according to an embodiment. In FIGs. 18 to 20, the first feature is indicated as "Feature 1" and the third feature is indicated as "Feature 3". In FIGs. 18 to 20, the vertical axis represents the first feature (Feature 1) and the horizontal axis represents the third feature (Feature 3). These are both quantities that have an angle [degree] dimension. FIGs. 18 to 20 plot the dispersion of the data extracted by expression (1) and expression (3) in a situation in which an occupant is seated at the position P3 where the driver-side rear seat is located and/or the position P4 where the passenger-side rear seat is located, which are positions close to one another. FIG. 19 illustrates the results for the case where an occupant is seated only in the right rear seat, that is, at the position P3 where the driver-side rear seat is located. FIG. 18 illustrates the results for the case where an occupant is seated only in the left rear seat, that is, at the position P4 where the passenger-side rear seat is located. FIG. 20 illustrates the results for the case where occupants are seated in both the right rear seat and the left rear seat, that is, at both the position P3 where the driver-side rear seat is located and the position P4 where the passenger-side rear seat is located. FIGs. 18 to 20 demonstrate that the first feature and the third feature can be used to distinguish between an occupant seated at the position P3 where the driver-side rear seat is located and an occupant seated at the position P4 where the passenger-side rear seat is located.

In the situation in which the electronic device 1 is installed as illustrated in FIGs. 9 and 10, the right rear seat (driver-side rear seat) and the left rear seat (passenger-side rear seat) may be located in proximity to one another, such that the distances from the electronic device 1 differ by about 5 cm to 10 cm. However, as illustrated in FIGs. 18 to 20, the signal processing unit 10 can use Feature 1 as a basis for separating (distinguishing) between the pattern of the state in which an occupant is seated only in the right rear seat (FIG. 19) and the pattern of the state in which an occupant is seated only in the left rear seat (FIG. 18). As illustrated in FIGs. 18 to 20, the signal processing unit 10 can use Feature 3 as a basis for separating (distinguishing) between the pattern of the state in which occupants are seated in both the right rear seat and the left rear seat (FIG. 20) and other patterns (FIG. 18 and/or FIG. 19).

The third feature (Feature 3) enables separation of the pattern of the state in which occupants are seated in both the right rear seat and the left rear seat (FIG. 20) for the following reason. The result of the angle-of-arrival estimation indicates the angle of the wave source with the strongest detected intensity. The detected intensity of the non-zero velocity component detected by the radar (the portion other than that described as the component corresponding to zero velocity in FIG. 11) changes with the respiratory state of the occupant, and the detected intensity when the occupant has completely stopped breathing is very small. Because of this, in the pattern of the state in which occupants are seated in both the right rear seat and the left rear state, a difference between the respiratory states of the two occupants will cause the angle of arrival to wobble between the angles of the two seats. This wobble can be quantified by using Feature 3. Consequently, as illustrated in FIGs. 18 to 20, the pattern in which only one occupant is present and the pattern in which multiple occupants are present can be separated.

Once the features are calculated in step S18, the signal processing unit 10 determines whether or not feature data exists for all arrangement patterns of an occupant or occupants seated in the seats in the cabin of the automobile (step S19). For example, in the case of an automobile with a seat located at each of the positions P1 to P2 as illustrated in FIG. 10, there are 16 possible patterns of an occupant or occupants seated in the seats, including the pattern with no occupants seated. In step S19, the signal processing unit 10 repeats the operations from step S11 to step S18 until feature data is acquired for all such patterns of an occupant or occupants seated in the seats. In the present embodiment, the user themself may change the locations of seats where an occupant or occupants are seated. In the present embodiment, the user may manually record an indication of which data corresponds to which locations of seats.

Upon determining in step S19 that feature data exists for all arrangement patterns, the signal processing unit 10 may create a model by machine learning (step S10) and end the operations illustrated in FIG. 12. In step S20, the signal processing unit 10 creates a model using machine learning by generating a table (see FIG. 16) of features in step S18 for all arrangement patterns in the cabin where the electronic device 1 is installed. In step S20, the signal processing unit 10 may generate a table as illustrated in FIG. 16 by repeating the process from step S11 to step S18 for all arrangement patterns in the cabin where the electronic device 1 is installed. In step S20, the signal processing unit 10 may generate a model through machine learning by adopting a support-vector machine (SVM) using a cubic polynomial kernel function, for example.

FIG. 21 is a flowchart for describing operations by the electronic device 1 according to an embodiment. FIG. 21 may illustrate operations for (2) actually distinguishing the seating of an occupant by the electronic device 1 according to an embodiment, which is performed on the basis of (1) the machine learning result generated on the basis of supervisory data as described above. That is, the operations illustrated in FIG. 21 may be for actually distinguishing the seating of an occupant and the seating position of the occupant by an electronic device 1 according to an embodiment.

The operations from step S31 to step S38 illustrated in FIG. 21 may be the same and/or similar to the operations from step S11 to step S18 described in FIG. 12. In step S39, the signal processing unit 10 uses the machine learning model generated in step S20 of FIG. 12 to distinguish the arrangement pattern of an occupant or occupants seated in the cabin, on the basis of the features extracted in step S38. For example, the signal processing unit 10 can obtain one predicted value of an occupant arrangement pattern per set of the 3n features illustrated in FIG. 16.

The arrangement pattern of seats where an occupant or occupants are seated that is distinguished in step S38 may be displayed on a display unit or the like of the external device 60, for example. For example, the signal processing unit 10 may display an image like the one illustrated in FIG. 10 on the display unit of the external device 60. In this case, upon determining that an occupant is seated in any of the positions P1 to P4, the signal processing unit 10 may, for example, change the display appearance of any of the positions P1 to P4 to indicate to the user that an occupant is seated. The signal processing unit 10 may also use at least one from among visual information, auditory information, and/or tactile information of any kind to indicate to the user the seat where an occupant is seated. The signal processing unit 10 may also utilize information indicating the seat where an occupant is seated in another process, or transmit such information to another device, another functional unit, or the like. For example, in an embodiment, another application may use the result of detecting the location of a seat where an occupant is seated as a basis for measuring the respiratory rate and/or heart rate of the occupant, issuing a warning about not wearing seatbelt, or the like.

According to an embodiment, the electronic device 1 can detect occupants seated in the seats located at the positions P1 to P4 in the cabin of an automobile as illustrated in FIG. 10, for example. Consequently, according to an embodiment, the electronic device 1 can transmit and receive radio waves to detect with good accuracy the presence and the presence position of a human or the like in an at least a partially enclosed space such as the cabin of an automobile, for example. In an embodiment, the electronic device 1 uses the temporal change in the angle-of-arrival estimation. According to the electronic device 1 as in an embodiment, occupants in the cabin of an automobile for example may be located in seats at a close distance to one another, but if the angles of the multiple persons present at the same distance with respect to the electronic device 1 are different, a temporal fluctuation occurs in the results of the angle-of-arrival estimation. According to an embodiment, the electronic device 1 can quantify this temporal fluctuation through acquisition of the variance or the like, combine the quantified fluctuation with information indicating the direction in which the absolute value of the angle is pointing, and thereby distinguish whether an occupant is present or absent in each seat.

In an embodiment, the estimation of the angle of arrival executed in the electronic device 1 is also expected to be affected by multipath. However, according to an embodiment, the electronic device 1 can utilize the features described above to create a machine learning model that also accounts for multipath, and thereby identify patterns of an occupant or occupants seated in the seats. The applicant conducted a demonstration experiment using a system including the electronic device 1 as described above, and confirmed that the system can distinguish patterns of an occupant or occupants seated in the seats with a high accuracy of 90% or more.

(Other embodiments)

The following describes other embodiments.

The electronic device 1 according to an embodiment is not limited to adopting millimeter-wave radar technology. For example, in an embodiment, the electronic device 1 can achieve the foregoing embodiment involving the FMCW scheme in the same and/or similar manner, even when adopting radio waves in the vicinity of millimeter waves, such as the centimeter-wave band or terahertz waves.

The foregoing embodiment gives an example in which, in step S20, a model is generated through machine learning by adopting a support-vector machine (SVM) using a cubic polynomial kernel function. However, in an embodiment, the signal processing unit 10 may also use another machine learning method, such as the k-nearest neighbors algorithm (KNN or k-NN) or a naive Bayes classifier.

In the foregoing embodiment, step S20 is described as extracting at least one from among the first, second, and third features. In an embodiment, in step S20, the signal processing unit 10 may also generate a model through machine learning on the basis of only the third feature, for example. In an embodiment, in step S20, the signal processing unit 10 may also generate a model through machine learning on the basis of the first feature and the third feature, for example. In an embodiment, in step S20, the signal processing unit 10 may also generate a model through machine learning on the basis of all of the first feature, the second feature, and the third feature, for example.

The foregoing embodiment describes, on the basis of the flowchart in FIG. 12, the generation of a feature table by performing one round of data acquisition for all seat arrangement patterns to be detected. However, in an embodiment, multiple rounds of data for all seat arrangement patterns to be detected may also be used. In an embodiment, data acquired after having changed the occupant or occupants seated in the seats and/or after having changed the posture (way of sitting) of an occupant or occupants seated in the seats may also be added to the table of features as illustrated in FIG. 16. This is thought to further increase the accuracy of distinguishing patterns of an occupant or occupant seated in the seats.

In the electronic device 1 illustrated in FIG. 2, the signal processing unit 10 is described as being provided with functions for performing various types of signal processing. However, in an embodiment, at least some of the processes to be performed by the signal processing unit 10 may also be performed by an external computer, processor, or the like, such as a cloud server, for example.

The present disclosure has been described on the basis of the drawings and examples, but note that a person skilled in the art could easily make various variations or revisions on the basis of the present disclosure. Consequently, it should be understood that these variations or revisions are included in the scope of the present disclosure. For example, the functions and the like included in each function unit may be rearranged in logically non-contradictory ways. Multiple function units or the like may be combined into one, or a function unit may be divided. Each embodiment according to the present disclosure described above is not limited to being carried out exactly according to each embodiment as described, and may be carried out by combining features or omitting some features, as appropriate. In other words, the content of the present disclosure enables a person skilled in the art to make various variations and revisions on the basis of the present disclosure. Therefore, these variations and revisions are included in the scope of the present disclosure. For example, in each embodiment, each function unit, means, step, and the like can be added to another embodiment or replaced by each function unit, means, step, and the like of another embodiment in logically non-contradictory ways. In each embodiment, multiple function units, means, steps, and the like can be combined into one, or each function unit, means, step, and the like can be divided. Each embodiment according to the present disclosure described above is not limited to being carried out exactly according to each embodiment as described, and can be carried out by combining features or omitting some features, as appropriate.

The embodiments described above are not limited solely to embodiments of the electronic device 1. For example, the embodiments described above may also be carried out as a method of controlling a device like the electronic device 1. Furthermore, the embodiments described above may also be carried out as a program to be executed by a device like the electronic device 1, for example, or as a storage medium or recording medium in which the program is recorded.

In the embodiments described above, the electronic device 1 is described as including components such as the transmitting antenna array 24 and the receiving antenna array 31 that form what is called a radar sensor. However, in an embodiment, the electronic device may be carried out as a configuration like the signal processing unit 10, for example. In this case, the signal processing unit 10 may be carried out as a unit having functions for processing signals handled by the transmitting antenna array 24, the receiving antenna array 31, and the like.

REFERENCE SIGNS

| | |
|---|---|
| 1 | electronic device |
| 10 | signal processing unit |
| 11 | signal generation processing unit |
| 12 | received signal processing unit |
| 21 | transmission DAC |
| 22 | transmission circuit |
| 23 | millimeter-wave transmission circuit |
| 24 | transmitting antenna array |
| 31 | receiving antenna array |
| 32 | mixer |
| 33 | reception circuit |
| 34 | reception ADC |
| 50 | communication interface |
| 60 | external device |

**Claims**

1. An electronic device comprising a signal processing unit configured to detect an object on the basis of a transmission signal transmitted as a transmission wave and a received signal received as a reflected wave resulting from the transmission wave being reflected by the object, wherein
the signal processing unit is configured to extract, from a result of estimating an angle, with respect to the electronic device, of a human body to be detected on the basis of the transmission signal and the received signal over a prescribed time, two-dimensional data having a time direction and a velocity direction that correspond to a distance between the electronic device and the human body, and extract a prescribed feature on the basis of the two-dimensional data.

2. An electronic device comprising a signal processing unit configured to detect an object on the basis of a transmission signal transmitted as a transmission wave and a received signal received as a reflected wave resulting from the transmission wave being reflected by the object, wherein
the signal processing unit is configured to extract, from a result of estimating an angle, with respect to the electronic device, of a human body detected on the basis of the transmission signal and the received signal over a prescribed time, two-dimensional data having a time direction and a velocity direction that correspond to a distance between the electronic device and the human body, extract a prescribed feature on the basis of the two-dimensional data, and thereby determine whether a human is present or absent in the surroundings of the electronic device and/or a position where the human is present.

3. The electronic device according to claim 1 or 2, wherein
the signal processing unit is configured to use a quantification of change over time in a direction-of-arrival estimation result as the prescribed feature.

4. The electronic device according to claim 1 or 2, wherein
the signal processing unit is configured to extract an unbiased standard deviation in the velocity direction on the basis of the two-dimensional data as the prescribed feature.

5. The electronic device according to claim 1 or 2, wherein
the signal processing unit is configured to use a result of executing a two-dimensional Fourier transform process on the transmission signal and the received signal as a basis for estimating the angle, with respect to the electronic device, of a human body to be detected on the basis of the transmission signal and the received signal.

6. The electronic device according to claim 1 or 2, wherein
the signal control unit is configured to use a result of receiving the received signal from a plurality of receiving antennas as a basis for estimating the angle, with respect to the electronic device, of a human body to be detected on the basis of the transmission signal and the received signal.

7. The electronic device according to claim 1 or 2, wherein
the signal processing unit is configured to extract an average in the velocity direction on the basis of the two-dimensional data as the prescribed feature.

8. The electronic device according to claim 1 or 2, wherein
the signal processing unit is configured to extract an unbiased standard deviation in the time direction on the basis of the two-dimensional data as the prescribed feature.

9. The electronic device according to claim 1 or 2, wherein
the electronic device is located in an at least partially enclosed space.

10. A method of controlling an electronic device, the method comprising:

   detecting an object on the basis of a transmission signal transmitted as a transmission wave and a received signal received as a reflected wave resulting from the transmission wave being reflected by the object;
   extracting, from a result of estimating an angle, with respect to the electronic device, of a human body to be detected on the basis of the transmission signal and the received signal over a prescribed time, two-dimensional data having a time direction and a velocity direction that correspond to a distance between the electronic device and the human body; and

extracting a prescribed feature on the basis of the two-dimensional data.

**11.** A program causing an electronic device to execute a process comprising:

detecting an object on the basis of a transmission signal transmitted as a transmission wave and a received signal received as a reflected wave resulting from the transmission wave being reflected by the object;
extracting, from a result of estimating an angle, with respect to the electronic device, of a human body to be detected on the basis of the transmission signal and the received signal over a prescribed time, two-dimensional data having a time direction and a velocity direction that correspond to a distance between the electronic device and the human body; and
extracting a prescribed feature on the basis of the two-dimensional data.

FIG. 1

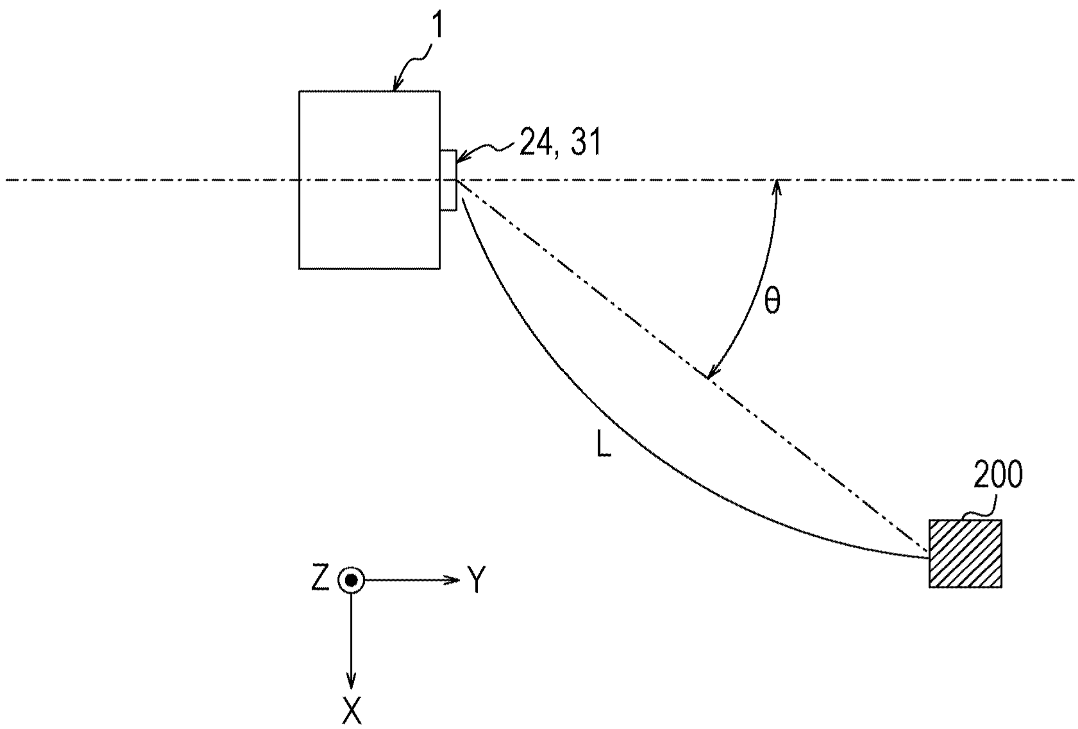

FIG. 2

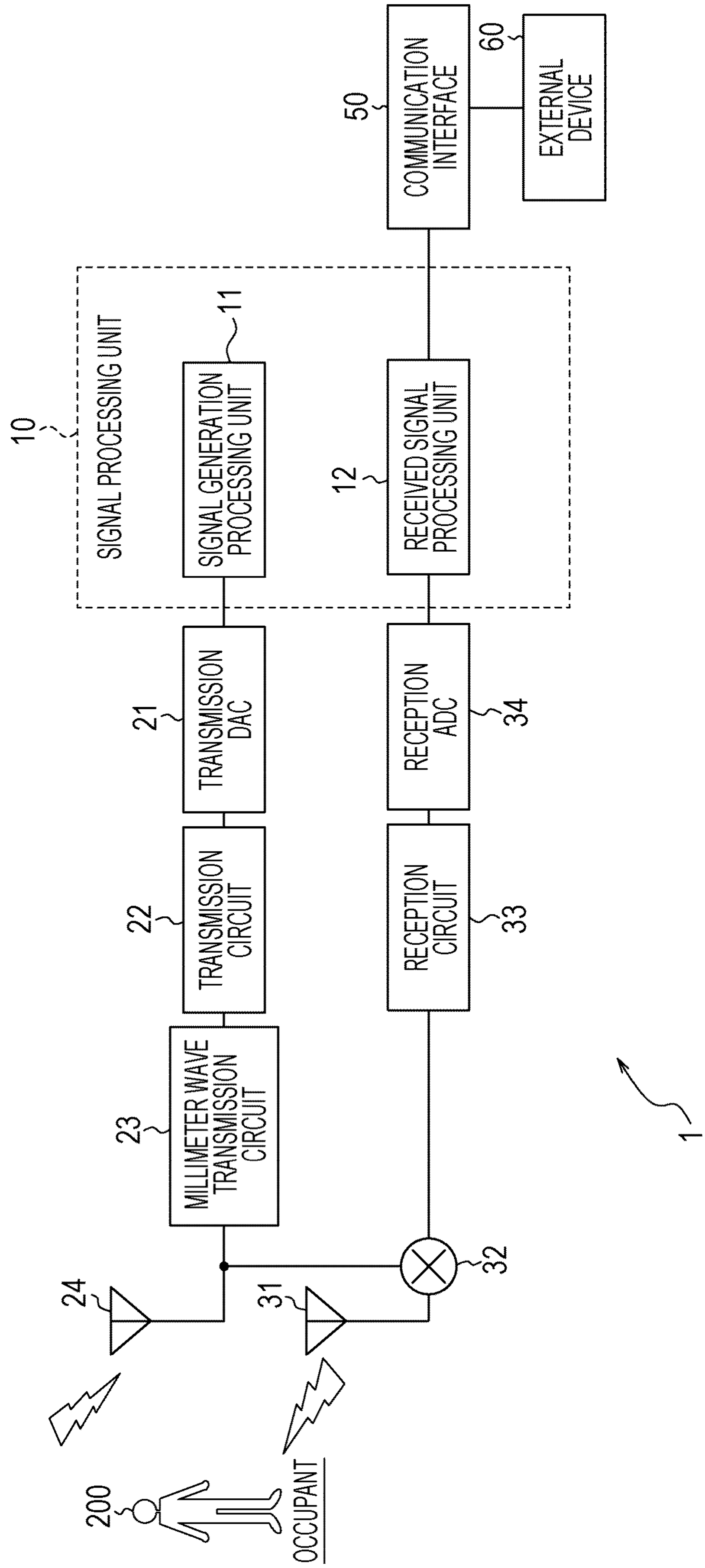
COMMUNICATION INTERFACE
50
EXTERNAL DEVICE
60
SIGNAL PROCESSING UNIT
10
SIGNAL GENERATION PROCESSING UNIT
11
RECEIVED SIGNAL PROCESSING UNIT
12
TRANSMISSION DAC
21
RECEPTION ADC
34
TRANSMISSION CIRCUIT
22
RECEPTION CIRCUIT
33
MILLIMETER WAVE TRANSMISSION CIRCUIT
23
24
31
32
200
OCCUPANT
1

FIG. 3

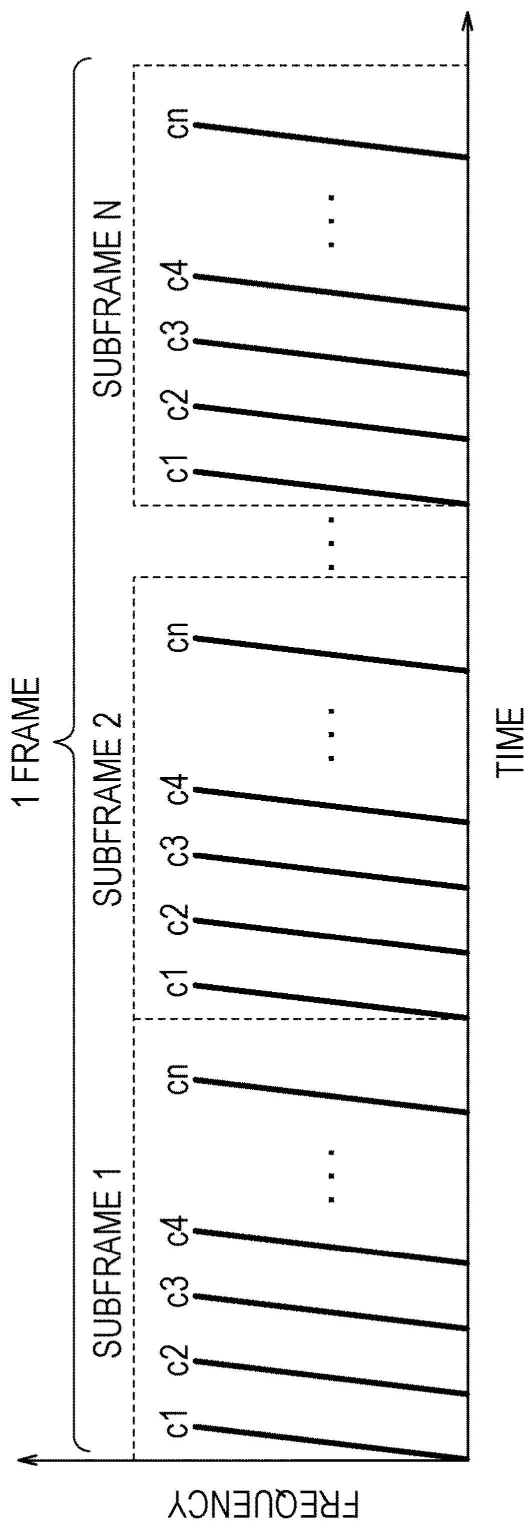
1 FRAME
SUBFRAME 1
SUBFRAME 2
SUBFRAME N
c1
c2
c3
c4
cn
TIME
FREQUENCY

FIG. 4

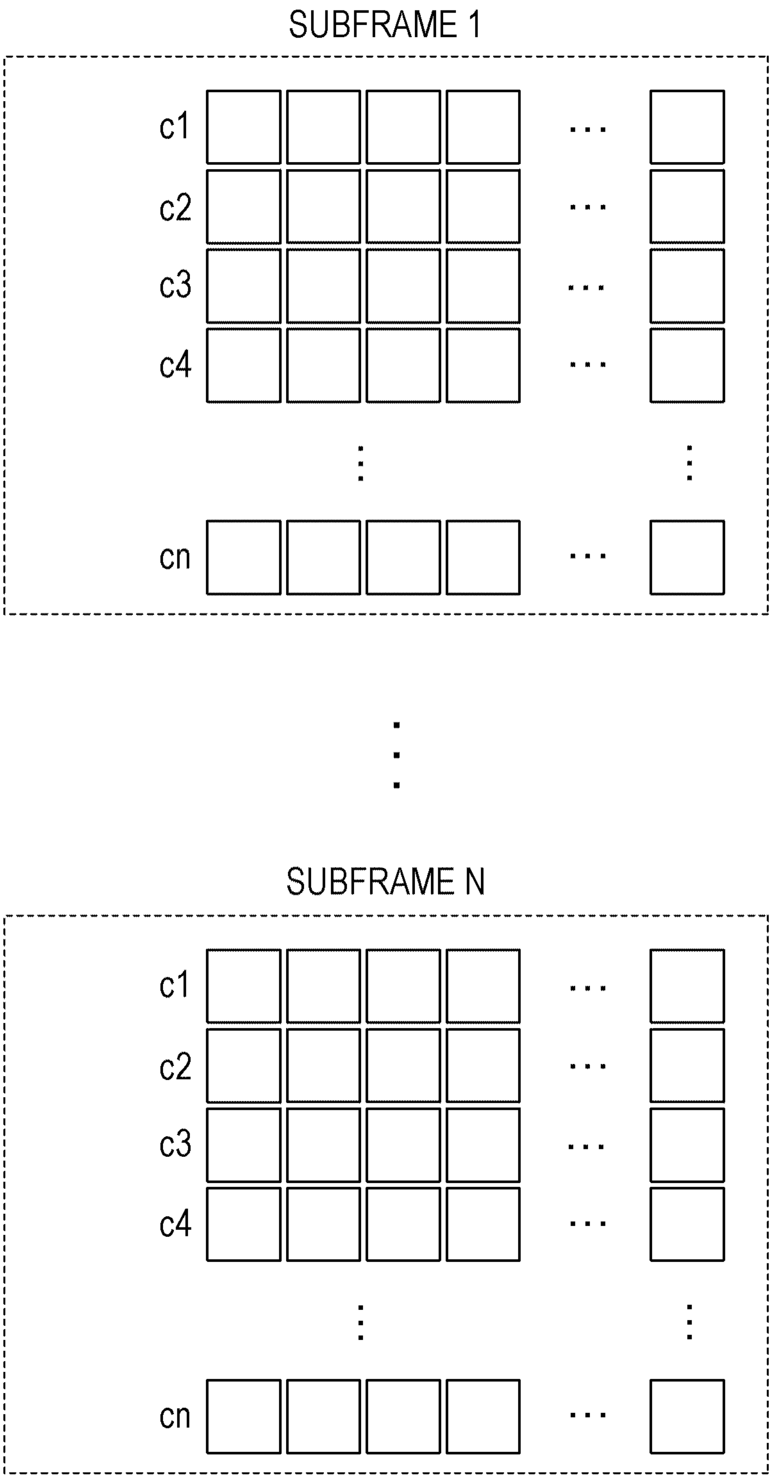

FIG. 5

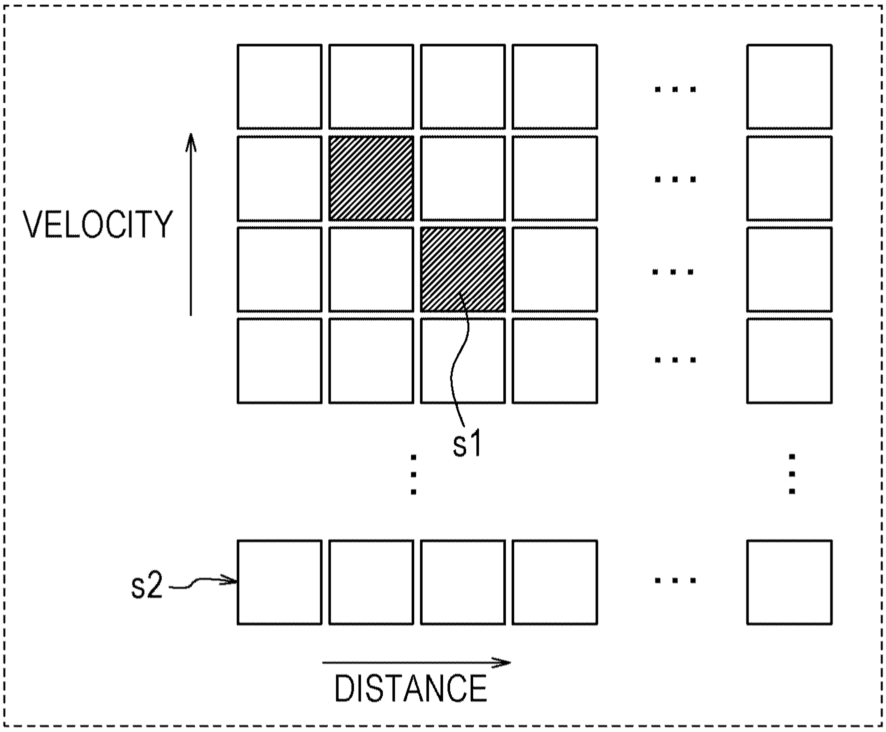

FIG. 6

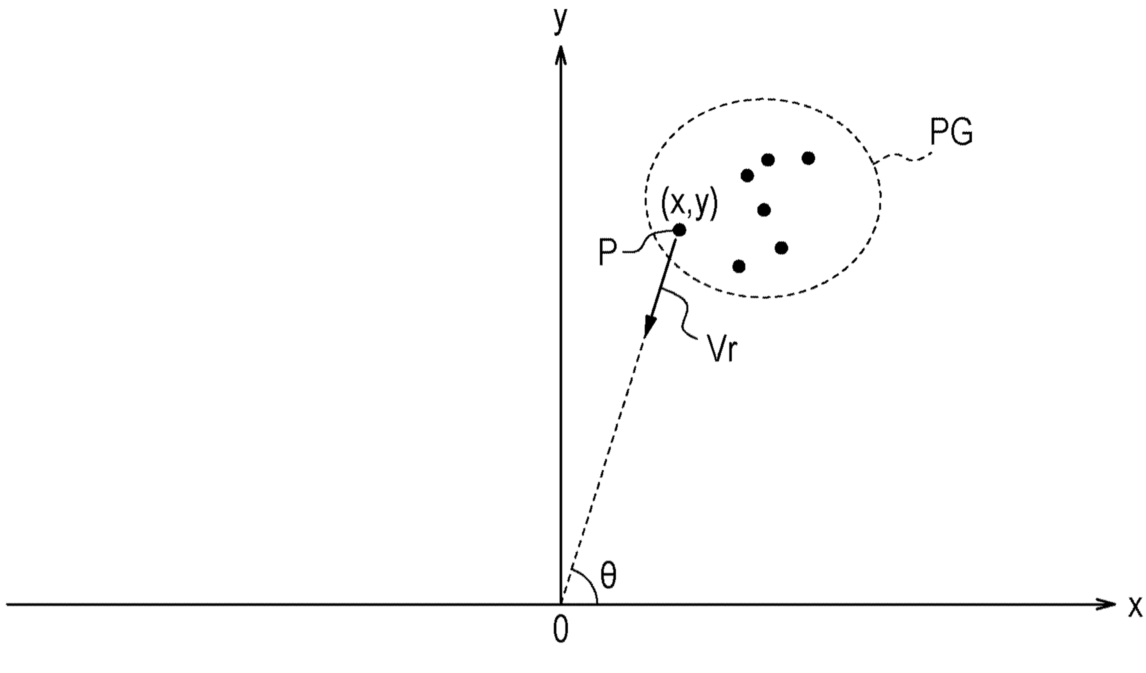

FIG. 7

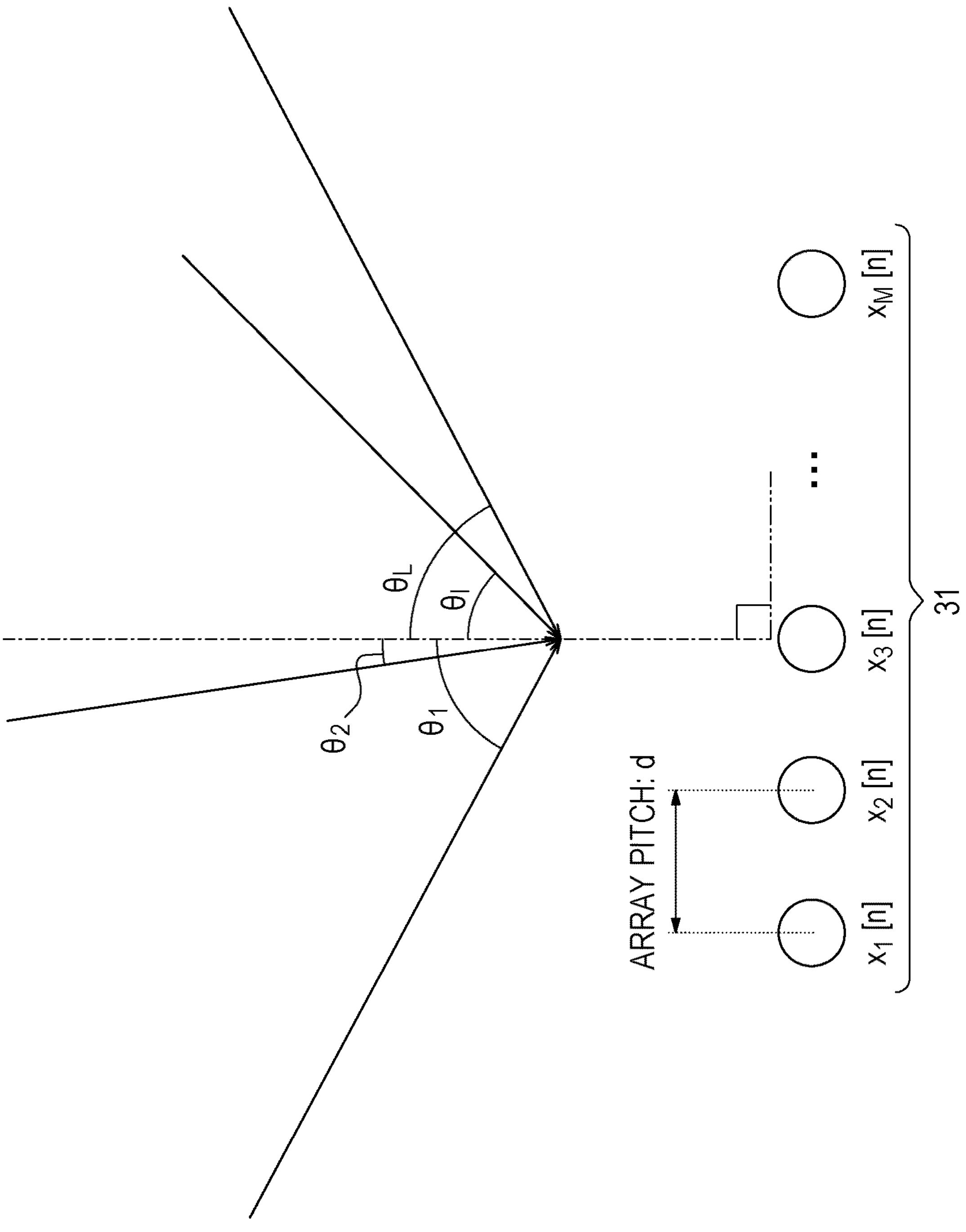

FIG. 8

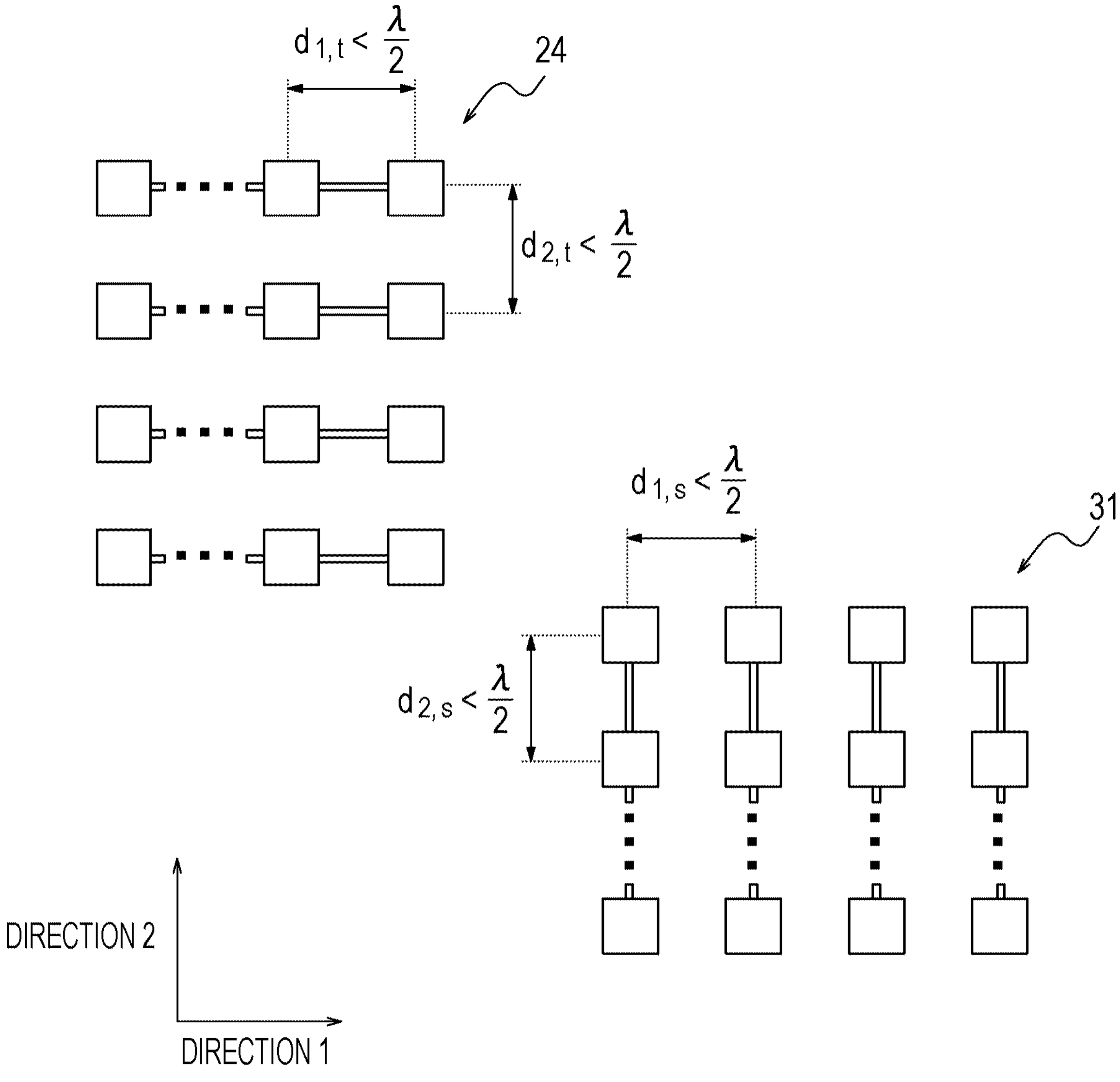
$d_{1,t} < \frac{\lambda}{2}$
24
$d_{2,t} < \frac{\lambda}{2}$
$d_{1,s} < \frac{\lambda}{2}$
31
$d_{2,s} < \frac{\lambda}{2}$
DIRECTION 2
DIRECTION 1

FIG. 9

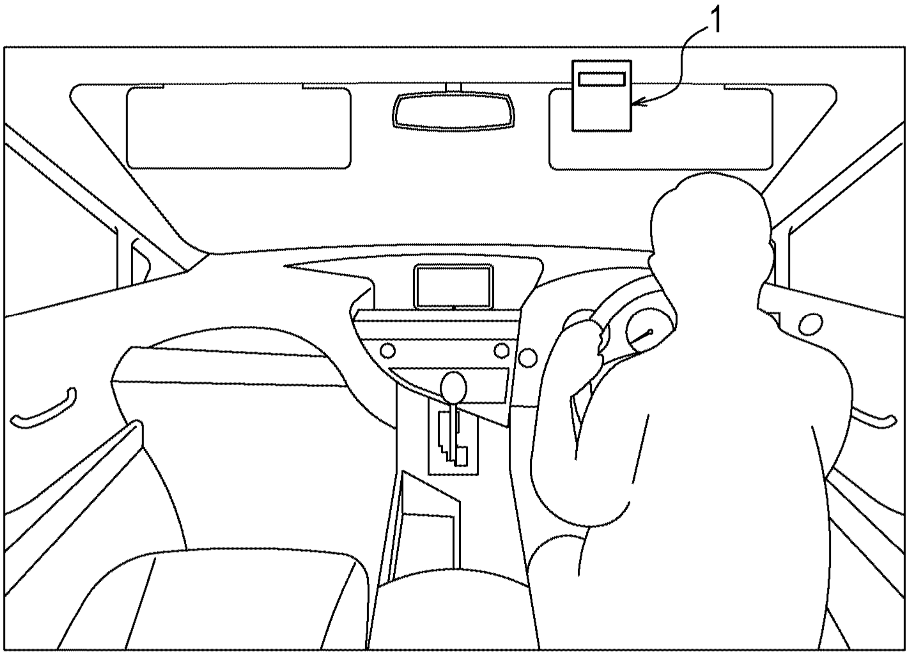

FIG. 10

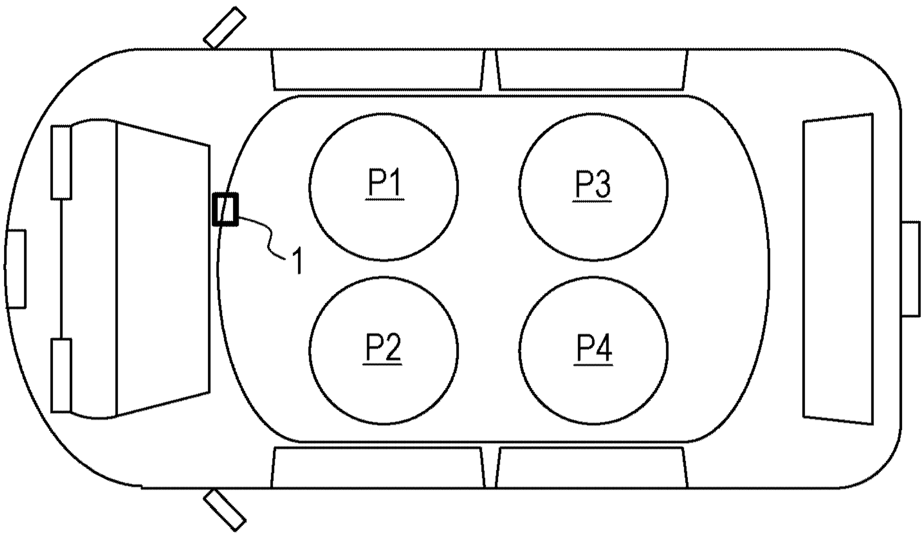

FIG. 11

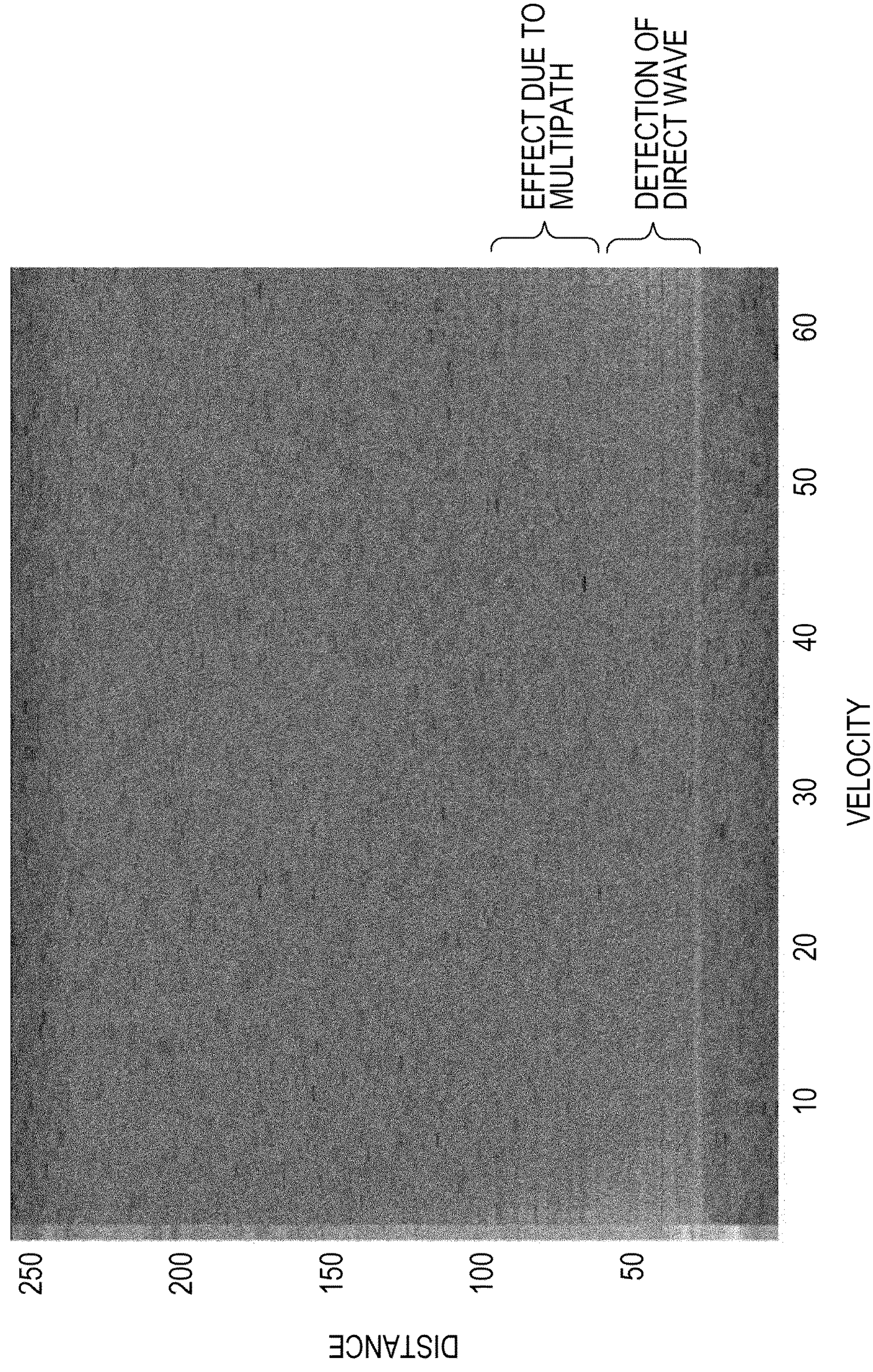
EFFECT DUE TO MULTIPATH
DETECTION OF DIRECT WAVE
DISTANCE
250
200
150
100
50
VELOCITY
10
20
30
40
50
60

FIG. 12

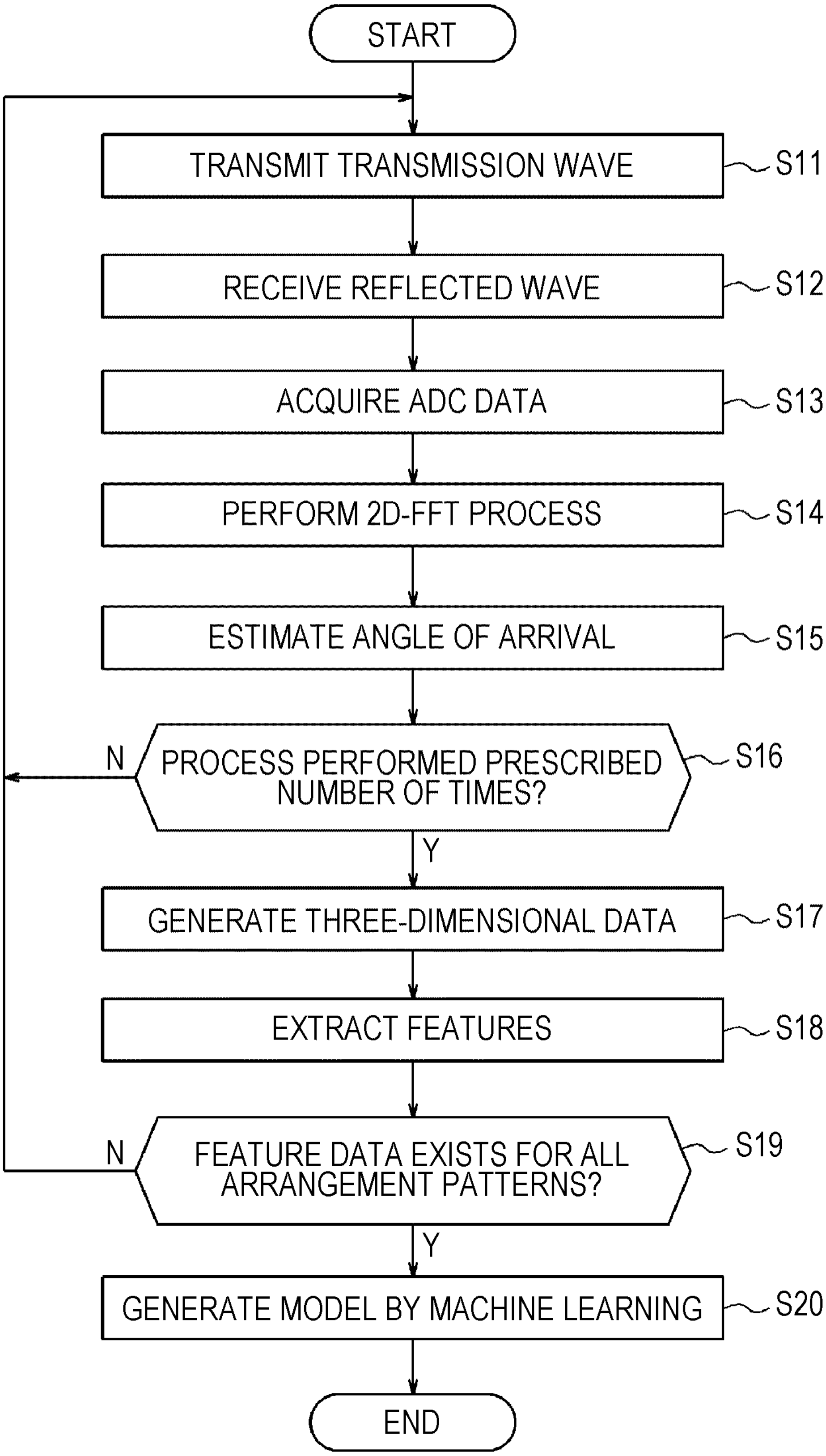
START
TRANSMIT TRANSMISSION WAVE
S11
RECEIVE REFLECTED WAVE
S12
ACQUIRE ADC DATA
S13
PERFORM 2D-FFT PROCESS
S14
ESTIMATE ANGLE OF ARRIVAL
S15
PROCESS PERFORMED PRESCRIBED NUMBER OF TIMES?
S16
N
Y
GENERATE THREE-DIMENSIONAL DATA
S17
EXTRACT FEATURES
S18
FEATURE DATA EXISTS FOR ALL ARRANGEMENT PATTERNS?
S19
N
Y
GENERATE MODEL BY MACHINE LEARNING
S20
END

FIG. 13

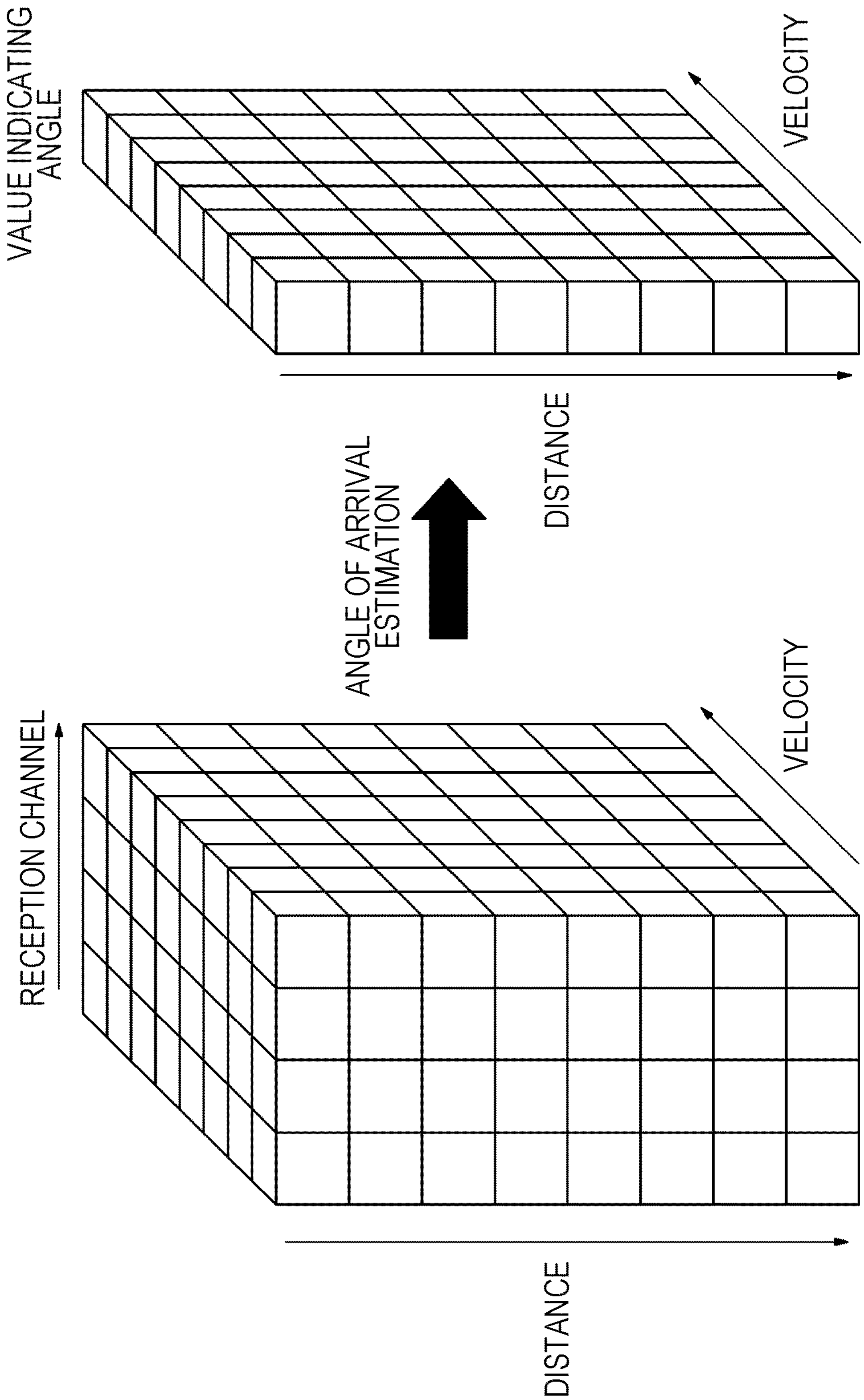
VALUE INDICATING ANGLE
VELOCITY
DISTANCE
ANGLE OF ARRIVAL ESTIMATION
RECEPTION CHANNEL
VELOCITY
DISTANCE

FIG. 14

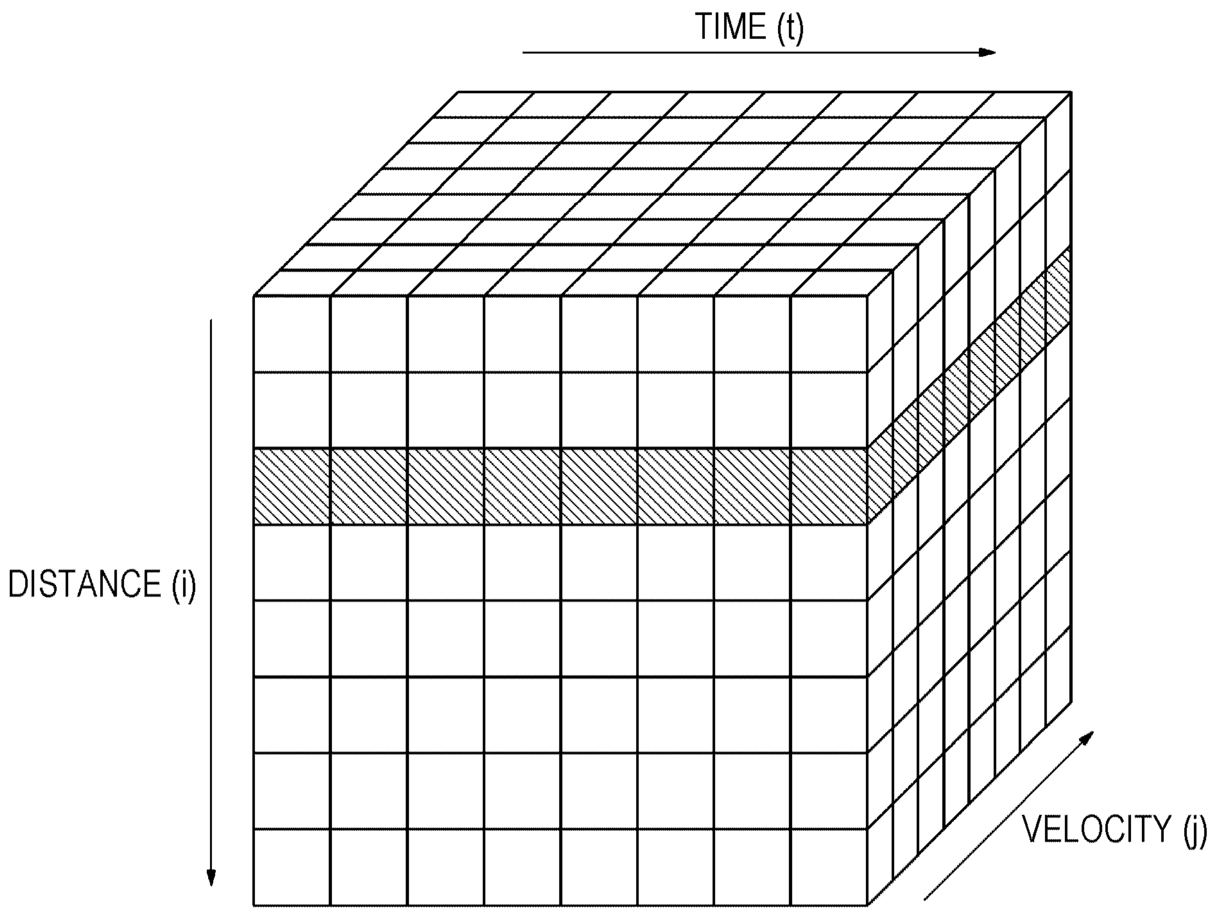

FIG. 15

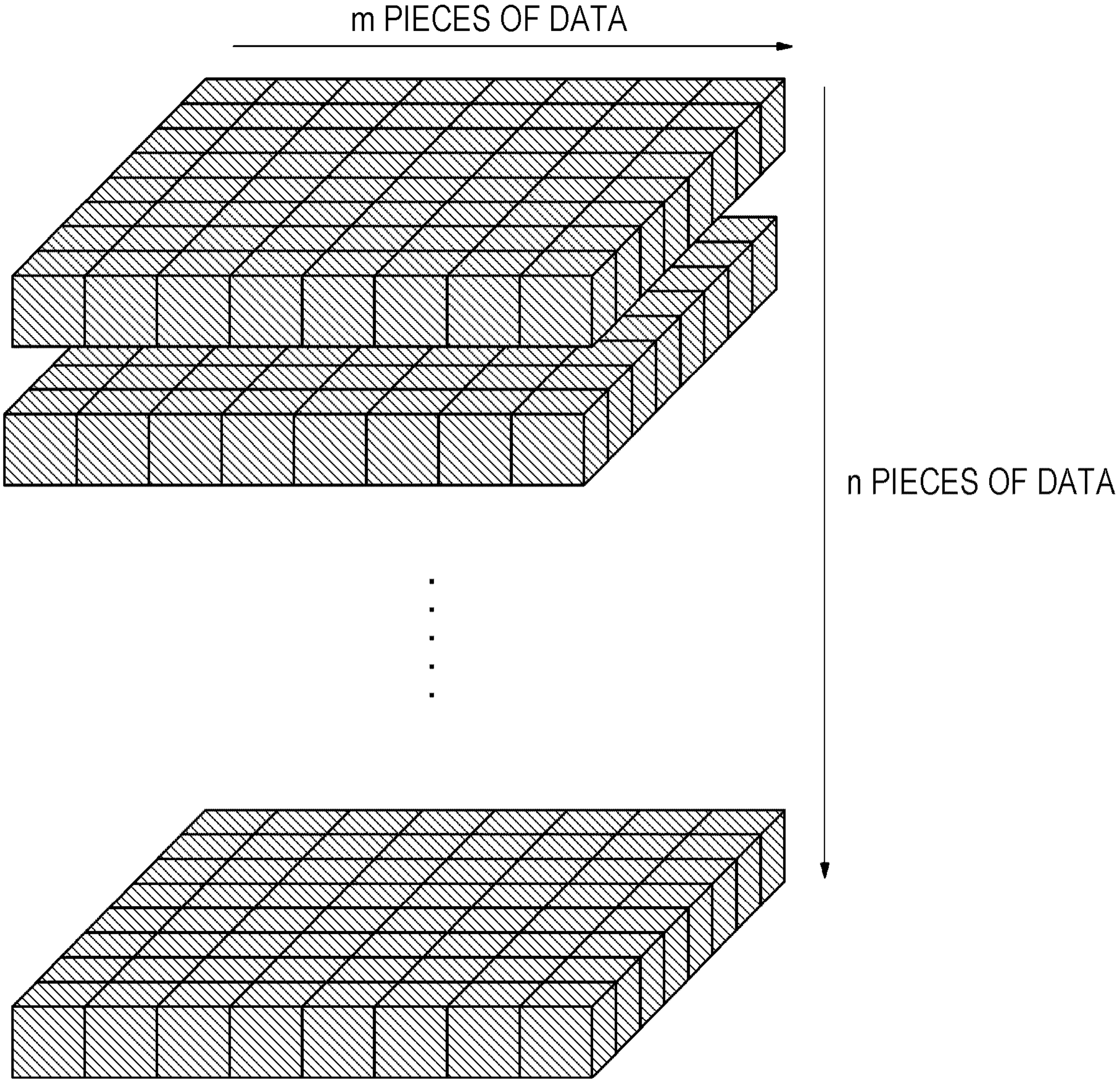

FIG. 16

| FEATURE 1 | FEATURE 2 | . . . | FEATURE 3n | |
|---|---|---|---|---|
| × | × | | × | DATASET 1 |
| × | × | | × | DATASET 2 |
| × | × | | × | DATASET 3 |
| | | | | |
| × | × | | × | DATASET m-1 |
| × | × | | × | DATASET m |

FIG. 17

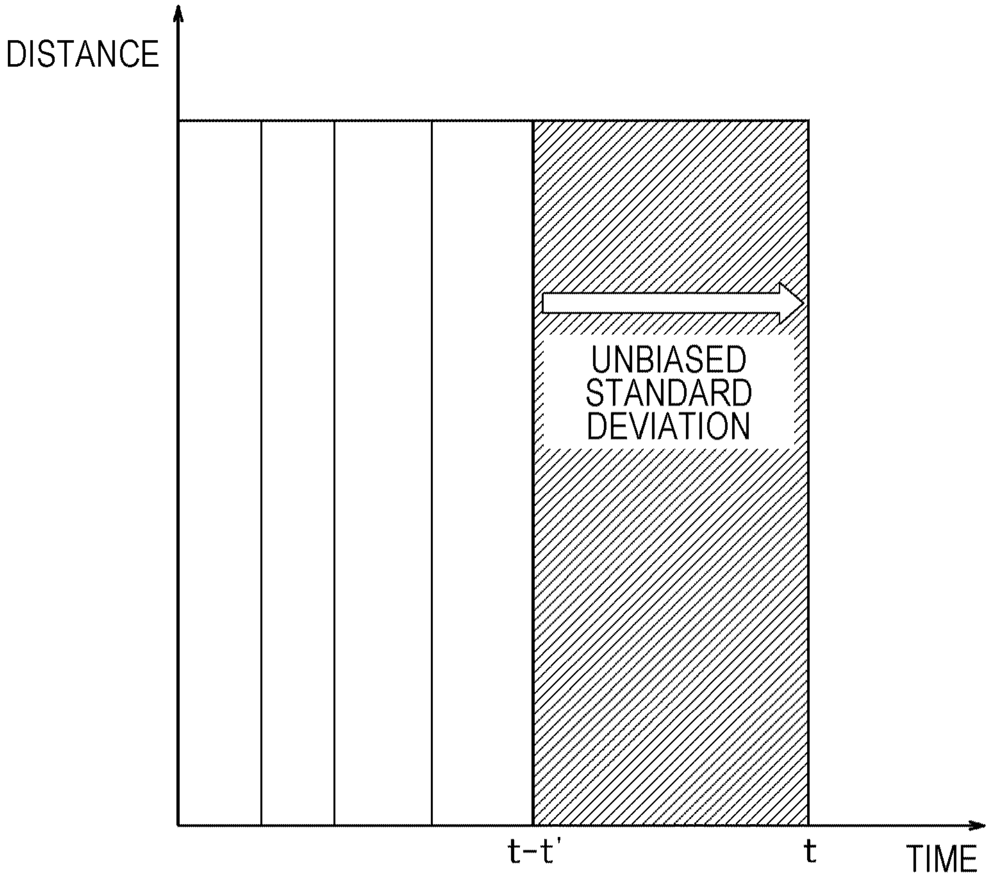

FIG. 18

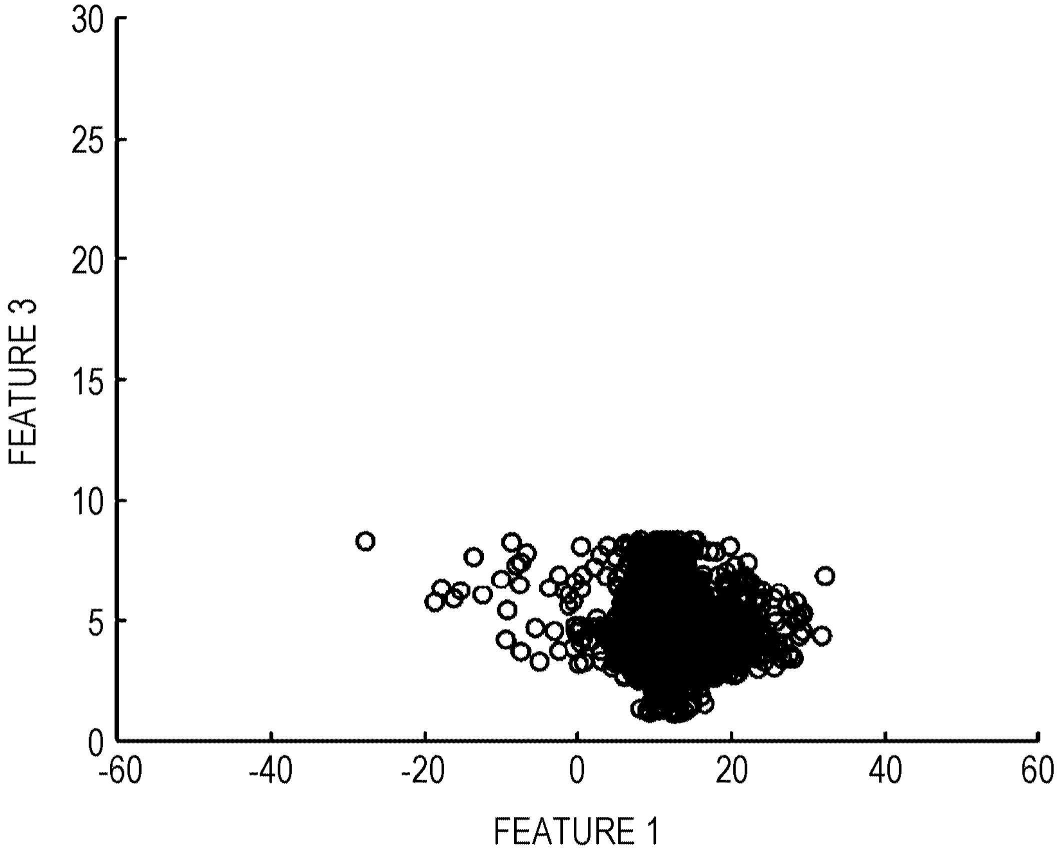

FIG. 19

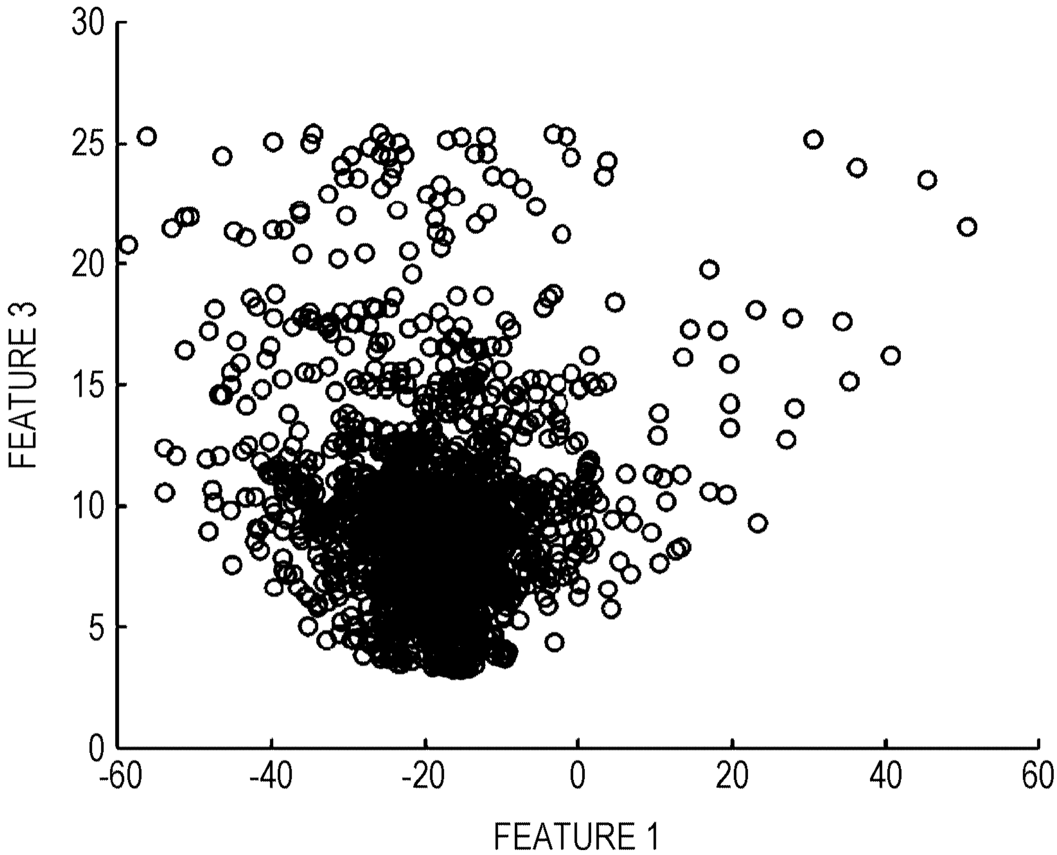

FIG. 20

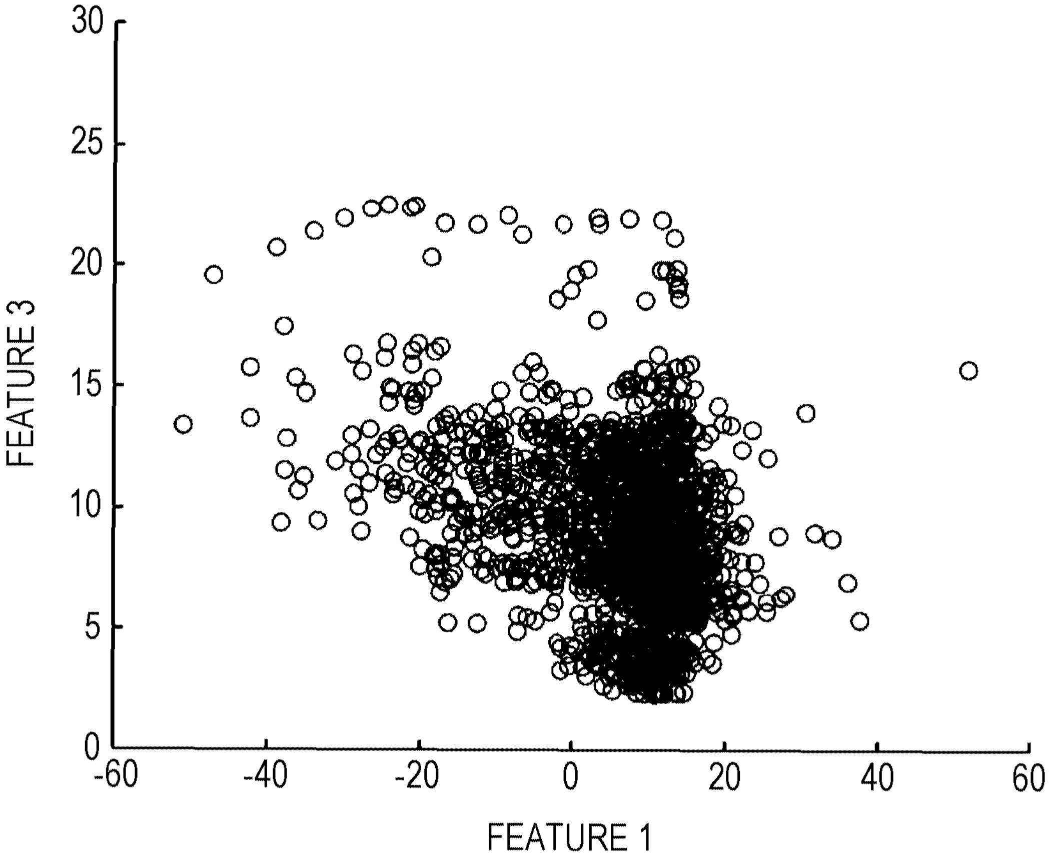

FIG. 21

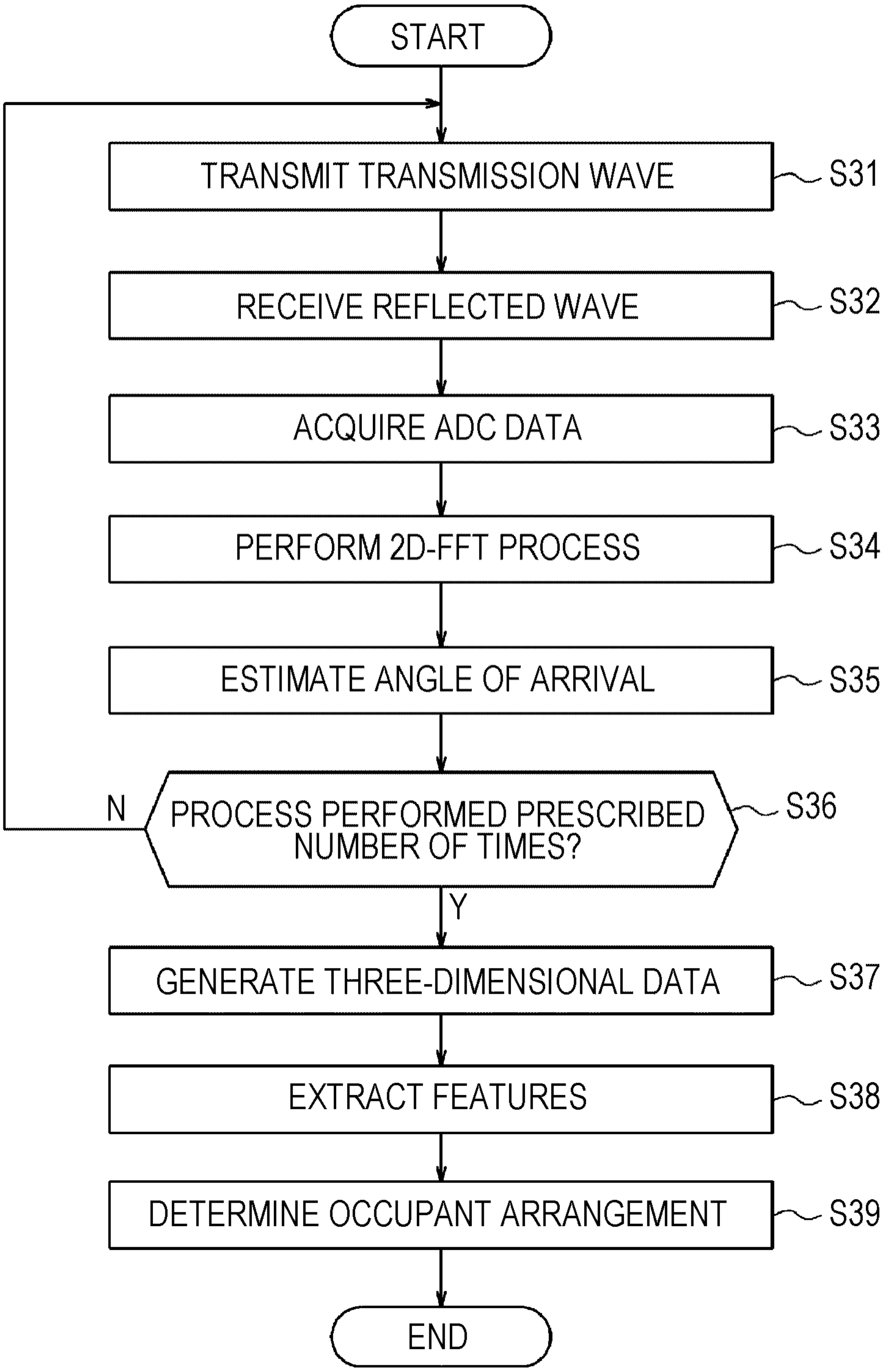
START
TRANSMIT TRANSMISSION WAVE
S31
RECEIVE REFLECTED WAVE
S32
ACQUIRE ADC DATA
S33
PERFORM 2D-FFT PROCESS
S34
ESTIMATE ANGLE OF ARRIVAL
S35
PROCESS PERFORMED PRESCRIBED NUMBER OF TIMES?
S36
N
Y
GENERATE THREE-DIMENSIONAL DATA
S37
EXTRACT FEATURES
S38
DETERMINE OCCUPANT ARRANGEMENT
S39
END

## INTERNATIONAL SEARCH REPORT

International application No.
**PCT/JP2024/007887**

**A. CLASSIFICATION OF SUBJECT MATTER**

***G01S 7/41***(2006.01)i; ***A61B 5/11***(2006.01)i; ***G01S 13/34***(2006.01)i; ***G01S 13/89***(2006.01)i
FI: G01S7/41; A61B5/11 110; G01S13/34; G01S13/89

According to International Patent Classification (IPC) or to both national classification and IPC

**B. FIELDS SEARCHED**

Minimum documentation searched (classification system followed by classification symbols)

G01S 7/00 - G01S 7/42; G01S 13/00 - G01S 13/95; A61B 5/06 - A61B 5/22; G01V 1/00 - G01V 99/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2021-1735 A (ASAHI KASEI ELECTRONICS CO., LTD.) 07 January 2021 (2021-01-07) paragraphs [0009]-[0131], fig. 1A-11B | 1-3, 5-6, 10-11 |
| Y | | 9 |
| A | | 4, 7-8 |
| X | US 2022/0381901 A1 (INFINEON TECHNOLOGIES AG) 01 December 2022 (2022-12-01) paragraphs [0065]-[0133], fig. 1-11 | 1-3, 5-6, 10-11 |
| Y | | 9 |
| A | | 4, 7-8 |
| X | US 2023/0014948 A1 (METIS IP (SUZHOU) LLC) 19 January 2023 (2023-01-19) paragraphs [0071]-[0099], fig. 4A-9 | 1-3, 5-6, 10-11 |
| Y | | 9 |
| A | | 4, 7-8 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 March 2024** | **02 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/007887**

**C. DOCUMENTS CONSIDERED TO BE RELEVANT**

| Category* | Citation of document, with indication, where appropriate, of the relevant passages | Relevant to claim No. |
|---|---|---|
| Y | JP 2022-188651 A (AISHIN KK) 21 December 2022 (2022-12-21) paragraphs [0023]-[0054], fig. 1-6 | 9 |
| A | WO 2012/020530 A1 (PANASONIC CORPORATION) 16 February 2012 (2012-02-16) entire text, all drawings | 1-11 |
| A | JP 2022-507777 A (KMB TELEMATICS, INC.) 18 January 2022 (2022-01-18) entire text, all drawings | 1-11 |
| A | JP 2022-76391 A (AISHIN KK) 19 May 2022 (2022-05-19) entire text, all drawings | 1-11 |

Form PCT/ISA/210 (second sheet) (July 2022)

**INTERNATIONAL SEARCH REPORT**
**Information on patent family members**

International application No.
**PCT/JP2024/007887**

| Patent document cited in search report | Publication date (day/month/year) | Patent family member(s) | Publication date (day/month/year) |
|---|---|---|---|
| JP 2021-1735 A | 07 January 2021 | (Family: none) | |
| US 2022/0381901 A1 | 01 December 2022 | EP 4099047 A1<br>CN 115436925 A | |
| US 2023/0014948 A1 | 19 January 2023 | WO 2021/174414 A1<br>CN 115244586 A | |
| JP 2022-188651 A | 21 December 2022 | US 2022/0397643 A1<br>paragraphs [0022]-[0053], fig. 1-6<br>DE 102022113806 A1 | |
| WO 2012/020530 A1 | 16 February 2012 | US 2012/0293359 A1<br>CN 102763001 A | |
| JP 2022-507777 A | 18 January 2022 | US 2020/0158861 A1<br>WO 2020/106849 A2<br>KR 10-2021-0093967 A<br>CN 113661412 A<br>CA 3120294 A1 | |
| JP 2022-76391 A | 19 May 2022 | US 2022/0146658 A1<br>DE 102021128801 A1<br>CN 114460658 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2023034884 A
- JP 2017181225 A


### Non-patent literature cited in the description


- **ALIZADEH MOSTAFA** ; **ABEDI HAJAR** ; **SHAKER GEORGE**. Low-cost low-power in-vehicle occupant detection with mm-wave FMCW radar. *IEEE SENSORS.*, 2019, 1-4